(19) Europäisches Patentamt
European Patent Office
Office européen des brevets

(11) EP 0 791 597 B1

(12) EUROPEAN PATENT SPECIFICATION

(45) Date of publication and mention of the grant of the patent:
**12.07.2000 Bulletin 2000/28**

(51) Int Cl.[7]: **C07D 501/06**, C07D 277/20, C07D 257/04, A61K 31/545

(21) Application number: **97301169.5**

(22) Date of filing: **21.02.1997**

# (54) Method for manufacture of cephalosporins and intermediates thereof

Verfahren zur Herstellung von Cephalosporinderivaten und Zwischenverbindungen

Procédé pour la préparation de céphalosporines et d'intermédiaires

(84) Designated Contracting States:
**DE FR GB IT**

(30) Priority: **21.02.1996 EP 96301161**

(43) Date of publication of application:
**27.08.1997 Bulletin 1997/35**

(73) Proprietor: **LUPIN LABORATORIES LIMITED**
**Bombay, Maharashtra - 400098 (IN)**

(72) Inventors:
- **Datta, Debashish, Lupin Lab. Ltd.**
  **462 046, Dist. Raisen, Madhya Predesh (IN)**
- **Rai, Bishwa Prakash, Lupin Lab. Ltd.**
  **462 046, Dist. Raisen, Madhya Predesh (IN)**
- **George, Vinod, Lupin Lab. Ltd.**
  **462 046, Dist. Raisen, Madhya Predesh (IN)**
- **Mehre, Kishor Gulabrao, Lupin Lab. Ltd.**
  **462 046, Dist. Raisen, Madhya Predesh (IN)**

(74) Representative:
**Harrison, David Christopher et al**
**MEWBURN ELLIS**
**York House**
**23 Kingsway**
**London WC2B 6HP (GB)**

(56) References cited:
**EP-A- 0 023 453**
**EP-A- 0 162 395**
**EP-A- 0 226 093**
**EP-A- 0 347 777**
**EP-A- 0 377 987**
**FR-A- 2 247 464**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to reactive derivatives of 2-(2-aminothiazol-4-yl)-(Z)-2-methoxyimino acetic acid; 1H-tetrazol-1-acetic acid and 2-(2-aminothiazol-4-yl)-(Z)-2-[(1-tertbutoxycarbonyl-1-methylethoxy)imino]acetic acid of the following general formula I.

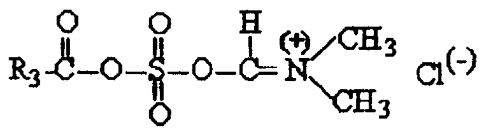

I

wherein

$R_3$ = 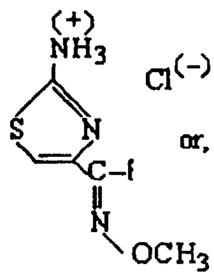 or, 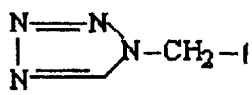 or, 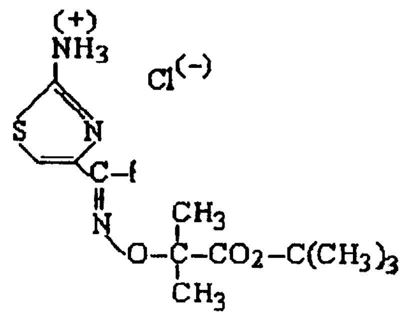

The present invention further relates to a process for the production of said reactive derivatives of general formula I as well as to a process for the manufacture of cephalosporin antibiotics using said reactive derivatives of general formula I.

It is known in the art to synthesize different cephalosporin antibiotics for instance compounds of formula II such as cefotaxime(IIa), ceftriaxone(IIb), cefazolin(IIc) and ceftazidime(IId), which generally comprise amidification of 7-position of appropriate cephalosporin nucleus with or without substitution at 3α-position, with the desired addendums.

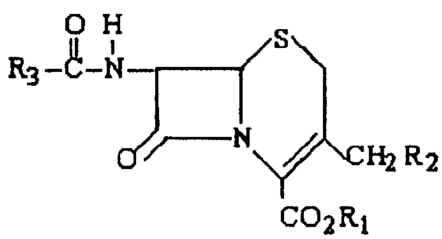

II

wherein $R_1$ = H, carboxylic protecting group for compounds IIa, IIb, IIc and IId

IIa : $R_2$ = acetoxy , $R_3$ = 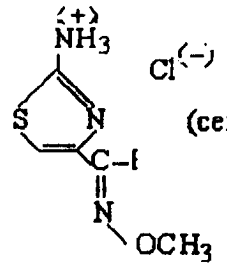 (cefotaxime)

IIb : $R_2$ = 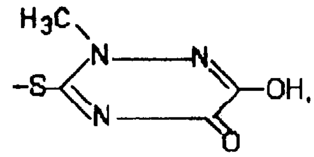 , $R_3$ = 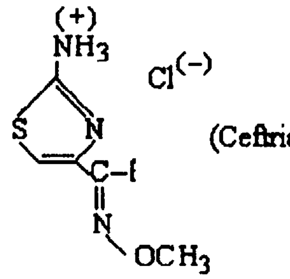 (Ceftriaxone)

IIc : $R_2$ = 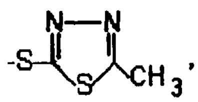 , $R_3$ = 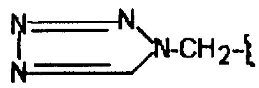 (Cefazolin)

IId : $R_2$ = 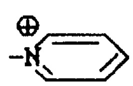 , $R_3$ = 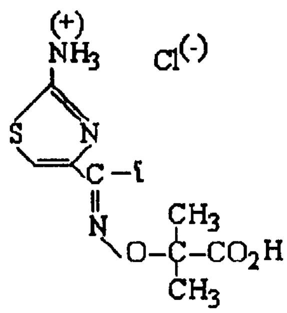 (Ceftazidime)

Usually, the synthesis by such known process of various cephalosporin antibiotics comprises acylation of 7-aminocephalosporanic acid (7-ACA) or 7-amino desacetoxycephalosporanic acid (7-ADCA) derivatives with a reactive derivative such as an acid chloride, an acid anhydride, an activated ester etc., of the addendum acid for instance of formulae III, IV and V.

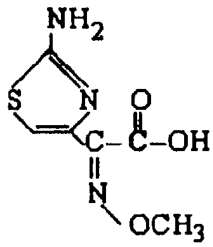

III

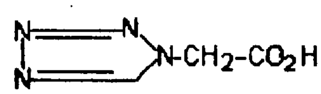

IV

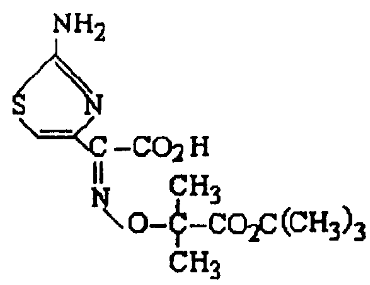

V

The above can be described schematically as follows:

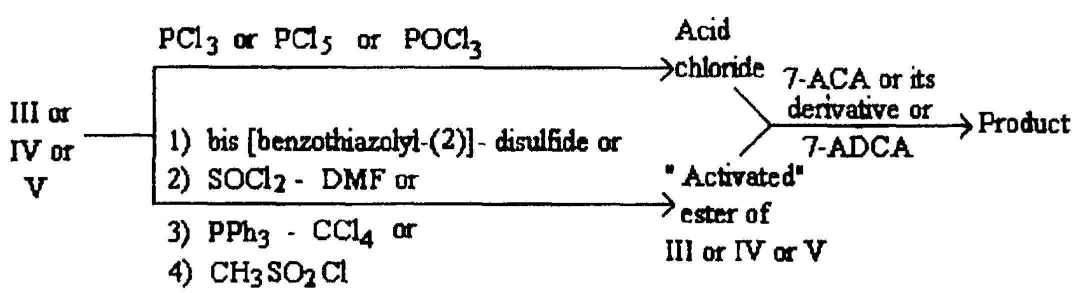


A number of methods utilising this concept has been reported for the functionalisation of position 7 of the desired cephalosporin nucleus and these are summarised below.

USP 4 152 432 describes acylation of 7-ACA with an acid chloride of formula VI in which the amino group in the thiazole ring is protected. The amino group is subsequently protected generally by hydrolysis or hydrogenolysis.

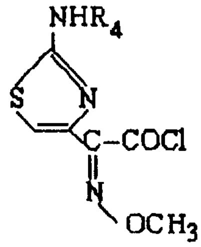

VI

wherein R4 = H or amino protecting group.

Because of the inherent inefficiency of amidification of the 7-amino group of cephalosporin nucleus and also of the steps involved in protection and deprotection of amino group of the thiazolyl ring and steps of purification of the final product, the overall yields are low and far from satisfactory.

Similar chemistry is employed in the preparation of ceftazidime (IId), a broad-spectrum cephalosporin antibiotic described in USP 4 258 041 and ceftazidime in its most stable pentahydrate form is described in USP 4 329 453.

Thus, USP 4 258 041 describes a process for the preparation of ceftazidime (IId) which comprises N-acylation of (6R,7R)-7-amino-3-(1-pyridiniummethyl)-3-cephem-4-carboxylic acid(VII) with 2-(2-tritylamino-thiazol-4-yl)-(Z)-2-[(1-tertbutoxycarbonyl-1-methylethoxy)imino]acetyl chloride(VIII) in a mixture of N,N-dimethylacetamide (DMAC) and acetonitrile to provide the ceftazidime intermediate i.e. (6R,7R)-7-[(Z)-2-(2-tritylaminothiazol-4-yl)-2-[(1-tertbutoxy carbonyl-1-methylethoxy)imino]acetamido]-3-(1-pyridiniummethyl)-3-cephem-4-carboxylic acid(IX) which was purified by crystallization as a DMF solvate followed by deprotection with formic acid to yield ceftazidime (IId). The process is schematically illustrated as follows

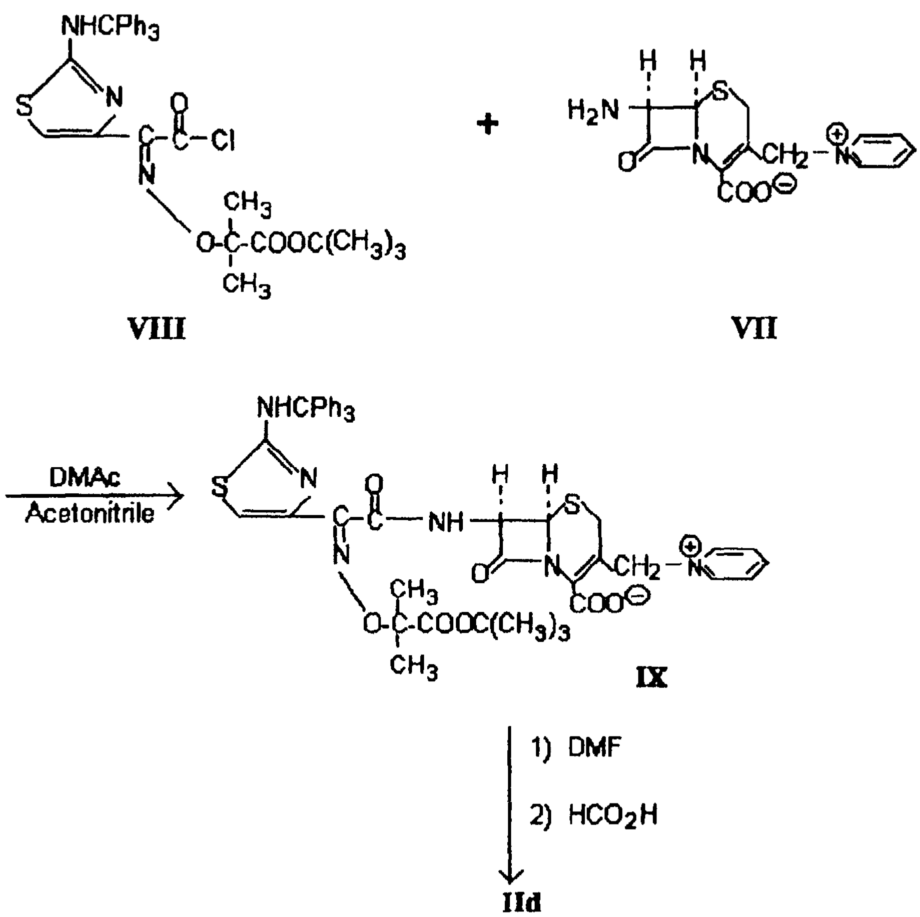


VIII + VII → IX → IId

SCHEME A

The same disadvantages as discussed above were suffered during this process.

EP 113 568 describes a method for the preparation of ceftazidime(IId) which is an improvement over the above method. The basic chemistry utilised in this process is same as above, however, the intermediate is directly crystallized out from the reaction mixture as a DMAC solvate.

EP 377 987 disclosed a method similar to above two methods involving protection and deprotection of the amino group of the thiazolyl ring. The only difference is the use of mixed anhydride of 2-(2-tritylaminothiazol-4-yl)-2-[(1-t-butoxycarbonyl-1-methylethoxy)imino]acetic acid with an alkyl or aryl sulfonic acid or a phosphoric acid.

Other patents which utilise similar chemistry are JP 52-102096; JP 54-157596 and GB 2 025 933 , which involve formation of 2-(2-aminothiazol-4-yl)-2-oxyimino acetyl chloride either with $PCl_3$, $PCl_5$, $SOCl_2$, or $POCl_3$.

Needless to say that the protection and deprotection of the amino group of the thiazolyl ring are involved in such conventional synthesis techniques for cephalosporin antibiotics.

Besides, protection and deprotection of the amino function, the acid chloride eg. 2-(2-aminothiazol-4-yl)-2-methoxyimino acetyl chloride, 1H-tetrazol-1-acetyl chloride and 2-(2-tritylamino-thiazol-4-yl)-2-[(1-tertbutoxy carbonyl-1-methylethoxy)imino] acetyl chloride are unstable creating further complication for the synthesis of the desired cephalosporin antibiotics.

USP 3 954 745 utilises the same concept i.e. formation of 1H-tetrazol-1-acetyl chloride in dimethyl acetamide and coupling the said acid chloride with DMF-solvate of the hydrochloride of 7-aminocephalosporanic acid derivative of the following formula X as shown below to yield cefazolin (Ic).

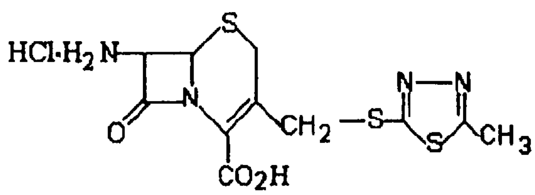

X

However, yields are low. Also, the acylating agent, 1H-tetrazol-1-acetyl chloride because of its inherent instability, is difficult to isolate and store at room temperature. Another limitation of this process is that acylation is to be carried out in strictly anhydrous conditions.

USP 5 317 099 describes a process for the synthesis of β-lactam derivatives such as cefotaxime, ceftriaxone in which silylated 7-ACA is acylated with acyloxy phosphonium chloride derivative of 2-(2-aminothiazol-4-yl)-2-(Z)-methoxyimino acetic acid which in turn is prepared from triphenyl phosphine(TPP), hexachloroethane or carbon tetrachloride and 2-(2-aminothiazol-4-yl)-(Z)-2-methoxyimino acetic acid.

Since, triphenyl phosphine (TPP) is used as a reactant, the overall cost of coupling the addendum III to the cephalosporin nucleus becomes high.

EP 037 380 describing a process for the synthesis of cefotaxime and ceftriaxone, and EP 282 531 describing a process for the synthesis of ceftazidime, utilise the chemistry involving acylation of 7-ACA derivatives with an intermediate of formula XI.

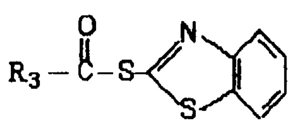

XI

wherein

XIa: $R_3$ = 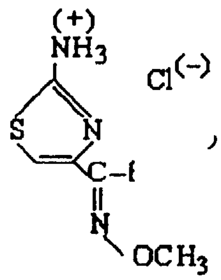 , XIb : $R_3$= 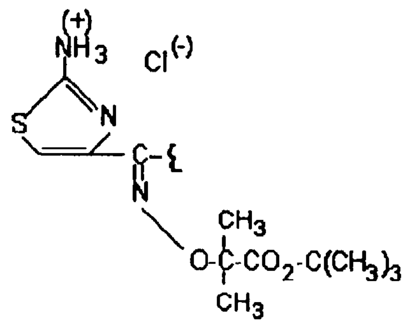

However, the synthesis of an appropriate oxyimino acetic acid S-benzothiazolyl ester of formula XI e.g 2-(2-aminothiazol-4-yl)-(Z)-2-(methoxyimino)thioacetic acid S-benzothiazolyl ester(MAEM)(XIa) and 2-(2-aminothiazol-4-yl)-2-(Z)-[1-tertbutoxy carbonyl-1-methylethoxy)imino]thioacetic acid S-benzothiazolyl ester (TAEM)(XIb) involves reaction of the corresponding oxyimino acetic acid with bis(benzothiazol-2-yl)disulfide and triphenyl phosphine (TPP).

WO 85/4659 utilises similar chemistry as above i.e. TAEM(XIb) is used as the reactive derivative which is coupled with 7-amino-3-(1-pyridiniummethyl)-3-cephem-4-carboxylate(VII). However, in this process ceftazidime tert butyl ester of formula XII is obtained only in amorphous form which is difficult to isolate.

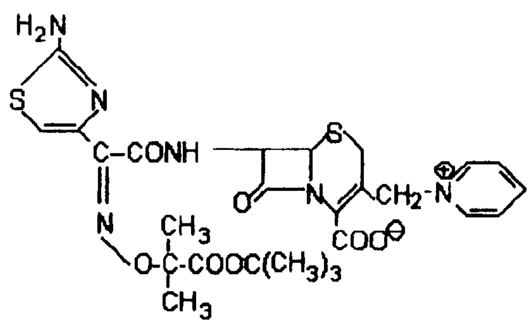

**XII**

Inspite of elagance of the chemistry employed, the use of costly reagent like triphenyl phosphine renders these processes very costly. Moreover, an additional step is required to remove the by-products resulted from the processes.

USP 5 037 988 describes a process for the production of cephalosporins in particular cefotaxime and ceftriaxone but not that of ceftazidime, in which an activated form of an organic acid i.e. 2-(2-aminothiazol-4-yl)-2-oxyimino acetyl sulfite dialkylformiminium halide hydrohalide of following formula (XIII);

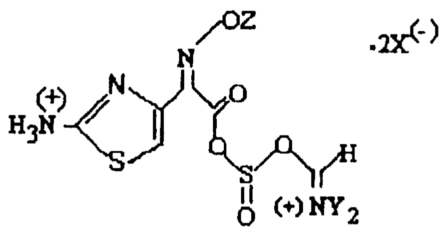 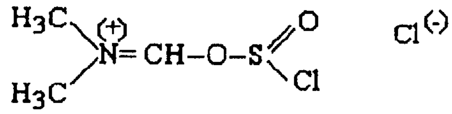

XIII XIV

is coupled with a 7-aminocephalosporanic acid derivative. The compound of formula XIII was prepared by reacting 2-(2-aminothiazol-4-yl)-2-oxyimino acetic acid with dimethyl formiminium chloride chlorosulfite of formula XIV which in turn was prepared by reacting approximately equimolar quantities of thionyl chloride and dimethylformamide at room temperature in specific solvents only like benzene or toluene, and hence a limitation.

However, poor results have been obtained when an attempt was made to prepare the compound of formula XIV in solvents such as chloroform and dichloromethane. In these solvents the compound XIV did not separate out in its characteristic form of insoluble oil, as the polarity of these solvents does not allow the formation of this reactant.

DE 2 921 316 describes a process for the manufacture of ceftazidime (IId) wherein (6R,7R)-3-acetoxy-methyl-7-amino-3-cephem-4-carboxylate is reacted with an appropriate N-protected thiazole acetic acid in the presence of dicyclohexyl carbodiimide in dimethylformamide to give an intermediate which was deblocked with trifluoroacetic acid followed by treatment with pyridine and sodium iodide to give ceftazidime sodium salt

Thus this process also involves the protection and deprotection of the amino group of the thiazolyl ring of the conventional processes, resulting in lowering of the overall yield of the final product.

Moreover, the process also involves the use of dicyclohexyl carbodiimide as a condensing agent which is toxic in nature.

Thus, it is evident that the procedures described in the prior art for the preparation of 7-acylamino cephalosporanic acid derivatives are either complex involving protection or deprotection or costly or have other limitations.

It is thus an object of the present invention to provide reactive derivatives of general formula I which would be suitable for use in the manufacture of cephalosporin antibiotics such as cefotaxime(IIa), ceftriaxone(IIb), cefazolin (IIc) and ceftazidime(IId).

Yet another object of the present invention is to provide a process for the synthesis of cephalosporin antibiotics such as cefotaxime, ceftriaxone, cefazolin and ceftazidime by which the protection and deprotection of the amino group of the thiazolyl ring reported in the known art may be avoided.

A further object of the present invention is to provide a process for the synthesis of cephalosporin antibiotics of the type mentioned above which may be more simply carried out and more cost effective than methods hitherto reported.

Yet another further object of the present invention is to provide for the synthesis of cephalosporin antibiotics of the type mentioned above which would favour better and consistent yield of cephalosporin antibiotics with desired characteristics for its use as an antibiotic.

Thus, according to one aspect of the present invention compounds of formula I,

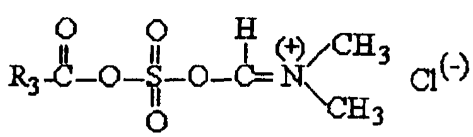

I

wherein

$R_3$ = 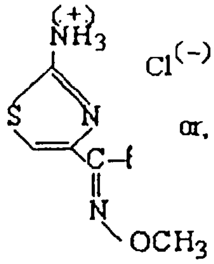 or, 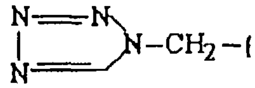 or, 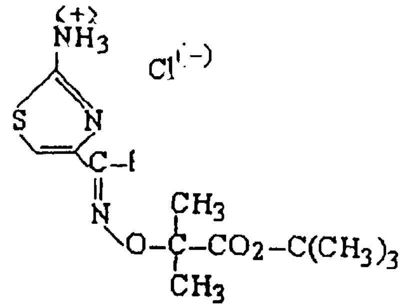

are provided by a process comprising reacting dimethyl formiminium chloride chlorosulfate (DFCCS) of formula XV,

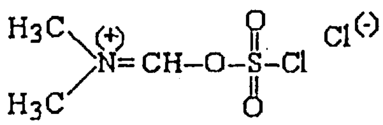

XV

with 2-(2-aminothiazol-4-yl)-(Z)-2 methoxyimino acetic acid (III) or 1H-tetrazol-1-acetic acid (IV) or 2-(2-aminothiazol-4-yl)-(Z)-2-[1-tertbutoxycarbonyl-1-methylethoxy)imino] acetic acid (V) in dichloromethane, chloroform, benzene or toluene at a temperature ranging from -30°C to -15°C to yield the compound of formula 1.

In the instance in formula I wherein

$R_3$ = 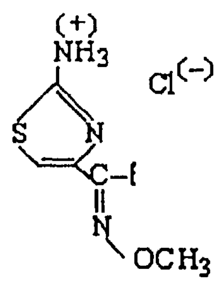

the above reactive derivative of formula I is a reactive derivative of 2-(2-aminothiazol-4-yl)-2-methoxyimino acetic acid of formula Ia,

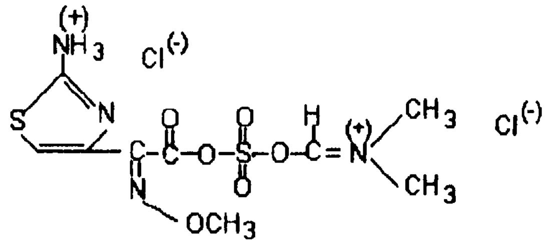

Ia

Further, in the instance in formula I,

$R_3$ = 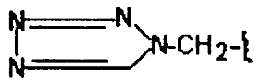

the above reactive derivative of formula I is a reactive derivative of 1H-tetrazol-1-acetic acid of formula 1b,

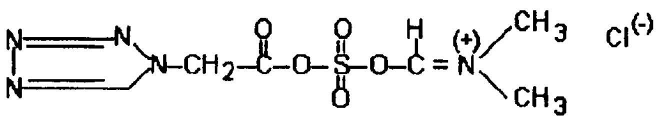

Ib

Further, in the instance, in formula I,

$R_3$ = 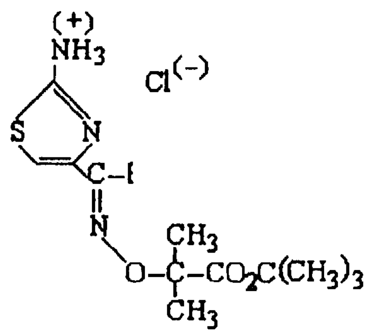

the above reactive derivative of formula I is a reactive derivative of 2-(2-aminothiazol-4-yl)-(Z)-2-[(1-tertbutoxycarbonyl-1-methylethoxy)imino)] acetic acid of formula Ic.

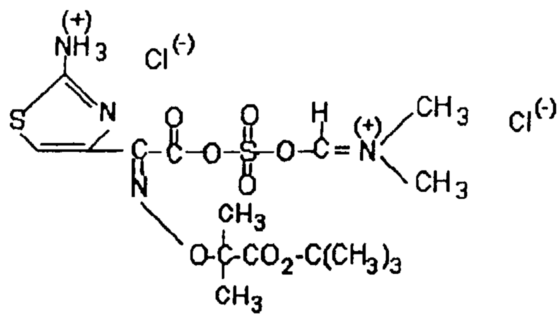

Ic

In accordance with the present invention the reactive derivative of 2-(2-aminothiazol-4-yl)-(Z)-2-methoxyimino acetic acid of formula Ia is obtained by activating 2-(2-aminothiazol-4-yl)-2-methoxyimino acetic acid (III) with N, N-dimethylformiminium chloride chlorosulfate (DFCCS) (XV) in dichloromethane, chloroform, benzene or toluene at a temperature ranging from -30°C to -15°C to yield reactive form 1a,

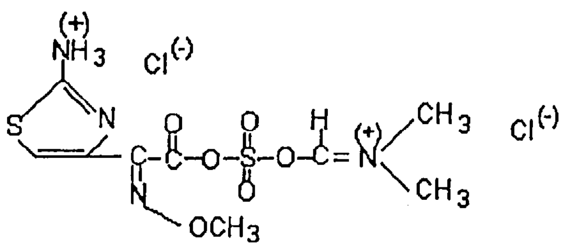

Ia

During the above process of manufacture of the reactive derivative of formula Ia and in particular during the said activation of 2-(2-aminothiazol-4-yl)-(Z)-2-methoxyimino acetic acid (III), the molar ratio of said DFCCS (XV) with respect to the said thiazolyl acetic acid (III) is 1.1 to 1.2 preferably 1.17.

Similarly, the reactive derivative of formula Ib is obtained by activating 1H-tetrazol-1-acetic acid (IV) with DFCCS (XV) in dichloromethane, chloroform, benzene or toluene at a temperature ranging from -30°C to - -15°C preferably at about -20°C to yield reactive form 1b.

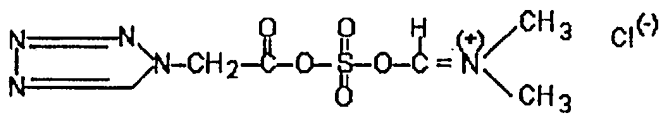

Ib

During the activation of 1H-tetrazol-1-acetic acid (IV), the molar ratio of said DFCCS (XV) to the said 1H-tetrazol-1-acetic acid (IV) is 1.1 to 1.2 preferably 1.17.

In accordance with the present invention the reactive derivative of 2-(2-aminothiazol-4-yl)-(Z)-2-[(1-tertbutoxycarbonyl-1-methylethoxy)imino] acetic acid of formula Ic is obtained by activating 2-(2-aminothiazol-4-yl)-(Z)-2-[(1-tertbutoxycarbonyl-1-methylethoxy)imino] acetic acid (V)

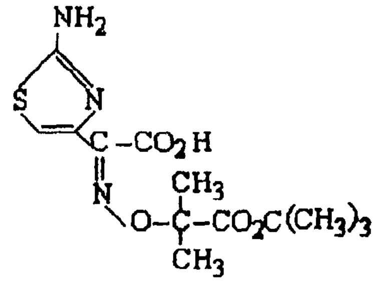

V

with N,N-dimethylformiminium chloride chlorosulfate (DFCCS) (XV) in dichloromethane at a temperature ranging from -20°C to -25°C to provide reactive derivative of formula Ic,

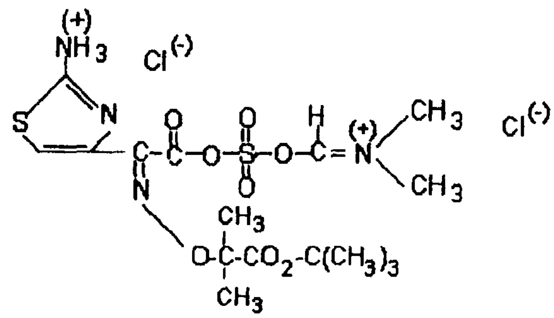

Ic

During the above process of manufacture of the reactive derivative of formula Ic and in particular during the said activation of 2-(2-aminothiazol-4-yl)-(Z)-2-[(1-tertbutoxycarbonyl-1-methylethoxy)imino] acetic acid(V), the molar ratio of DFCCS (XV) to the said thiazolyl acetic acid (V) was preferably maintained in the range of 1.0 to 1.3.

In accordance with a further aspect of the invention there is provided a process for the preparation of the desired cephalosporin antibiotics of general formula II which comprises reacting silylated 7-aminocephalosporanic acid derivatives of formula XVI,

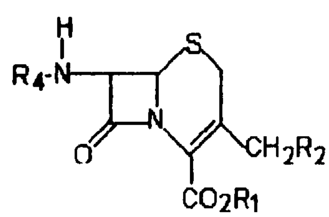

XVI

in which $R_1$ = carboxylic protecting group or hydrogen;

$R_2$ = acetoxy, 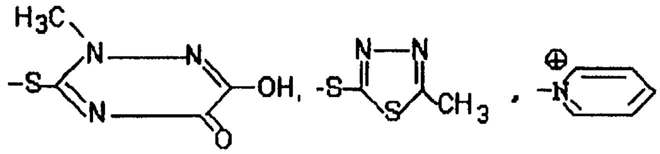

$R_4$ = silyl group or hydrogen.

with a reactive derivative of 2-(2-aminothiazol-4-yl)-(Z)-2-methoxyimino acetic acid of formula Ia or 1H-tetrazol-1-acetic acid of formula Ib or 2-(2-aminothiazol-4-yl)-(Z)-2-[(1-tertbutoxycarbonyl-1-methylethoxy)imino]acetic acid of formula Ic in dichloromethane at a temperature ranging from -70°C to -30°C.

Thus, in accordance with the present invention the process for the manufacture of cephalosporin antibiotics such as cefotaxime(IIa) and ceftriaxone(IIb) comprises activating 2-(2-aminothiazol-4-yl)-(Z)-2-methoxyimino acetic acid(III) with DFCCS (XV) in dichloromethane at a temperature ranging from -30°C to -15°C to yield the reactive derivative Ia, which is then coupled with silylated 7-aminocephalosporanic acid (XVI) at a temperature ranging from -70°C to -30°C preferably -55°C to yield the desired cephalosporins such as cefotaxime(IIa), ceftriaxone(IIb).

Similarly, cefazolin (IIc) is prepared by coupling silylated 7-amino-3-(2-methyl-1,3,4-thiadiazol-5-yl)thiomethyl-3-cephem-4-carboxylic acid and reactive derivative of 1H-tetrazol-1-acetic acid of formula Ib at a temperature ranging from -70°C to -30°C preferably -55°C to yield cefazolin(IIc).

In the case of ceftazidime(IId) the ceftazidime penultimate intermediate, ceftazidime tertbutyl ester(XII) is obtained by the process of the present invention and the intermediate (XII) is further converted to ceftazidime pentahydrate by following the procedure described in the prior art.

Thus, the process for the manufacture of ceftazidime of formula IId comprises,

a. silylating 7-amino-3-(1-pyridiniummethyl)-3-cephem-4-carboxylic acid of formula VII

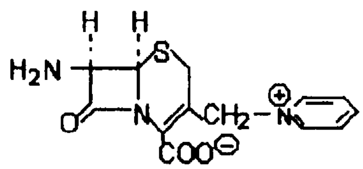

VII

with a silylating agent in the presence of an acid scavenging agent at a temperature ranging from 18°C to 25°C; to provide silylated 7-amino-3-(1-pyridiniummethyl)-3-cephem-4-carboxylic acid of formula (XVId);

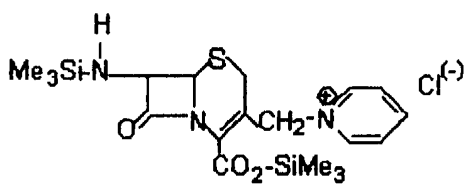

XVId

b. activating 2-(2-aminothiazol-4-yl)-(Z)-2-[(1-tertbutoxycarbonyl-1-methylethoxy)imino] acetic acid(V)

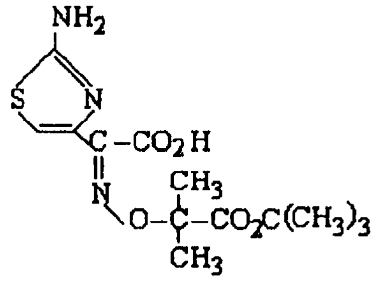

V

with N,N-dimethylformiminium chloride chlorosulfate (DFCCS) (XV),

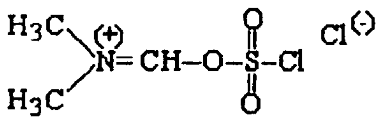

XV

in dichloromethane, chloroform benzene or toluene at a temperature ranging from -20°C to -25°C to obtain reactive derivative of formula Ic;

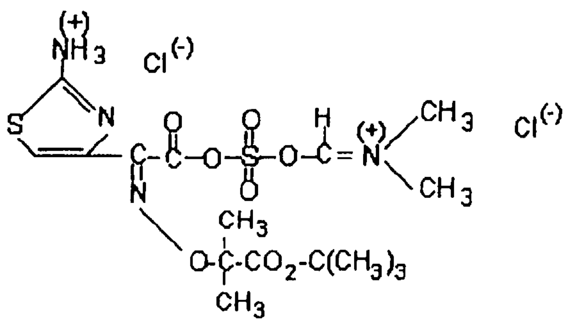

Ic

c. acylating the silylated 7-amino-3-(1-pyridiniummethyl)-3-cephem-4-carboxylic acid (XVId) of step (a) with the reactive derivative of formula Ic of steb (b) at a temperature ranging from -70°C to -60°C preferably -70°C to -65°C to obtain ceftazidime tertbutyl ester ( XII )

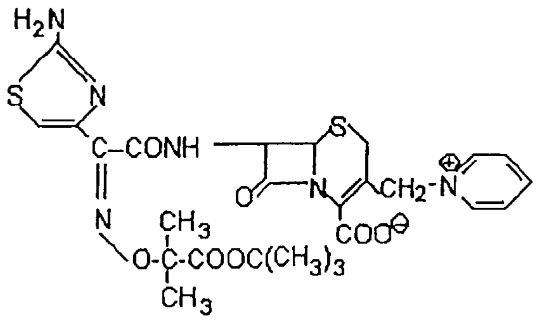

XII

which is converted to ceftazidime pentahydrate (IId) via ceftazidime dihydrochloride.

Thus, the above process for the manufacture of ceftazidime (IId) of the invention comprises reacting silylated 7-amino-3-(1-pyridiniumethyl)-3-cephem-4-carboxylic acid of formula XVId with reactive derivative of 2-(2-aminothiazol-4-yl)-(Z)-2-[(1-tertbutoxycarbonyl-1-methylethoxy)imino] acetic acid of formula Ic in dichloromethane at a temperature ranging from - 60°C to -70°C in the presence or absence of an acid scavenging agent to obtain ceftazidime tertbutyl ester (XII) which was converted to ceftazidime dihydrochloride following known procedure such as described in USP 4 258 041, which is further converted to its most stable pentahydrate form by fully known procedure as described in USP 4 329 453.

During the acylation reaction an acid scavenging agent can be optionally used. The preferred acid scavenging agent is N,N-dimethyl aniline and preferably the acid scavenging agent should be added to the silylated 7-amino-3-(1-pyridiniummethyl)-3-cephem-4-carboxylic acid(XVId) at -50°C.

During the process of the acylation reaction either the silylated 7-amino-3-(1-pyridiniummethyl)-3-cephem-4-carboxylic acid(XVId) can be added to the reactive derivative Ic or vice-versa. However, for better results the addition should be preferably completed within 5 to 15 minutes.

Thus, in accordance with the present invention DFCCS (XV) which is used for activating the addendum acids such as III, IV or V, is a known compound and described in the literature viz.Z.Chem, 6(4), 148(1966), J.C.S. Perkin Trans I, 2004-2007 (1972); Bull Chem Soc. Jpn, 58, 1063-1064; Adv. Org. Chem. 9(2), 5, (1979); Synthetic Reagents VoL 4, 388-389; Angew Chem International Edit, 1(12), 647 (1962).

While DFCCS (XV) prepared by any known process might be used but the inventors have found that best results are obtained when DFCCS (XV) is prepared by the following process.

The preferred process of obtain the DFCCS (XV) comprises adding sulfinyl chloride to dimethyl formamide at -20°C. The temperature is raised to 0°C at which the solid adduct crystallised out, which is vigorously stirred for one hour followed by addition of dichloromethane to the resulting reaction mixture. The temperature was raised to 15°C to 20°C and at this temperature the solid crystals melt resulting in the formation of immiscible layer of the desired adduct i.e. XV.

Such mode of preparation of DFCCS (XV) is illustrated in the following scheme B:

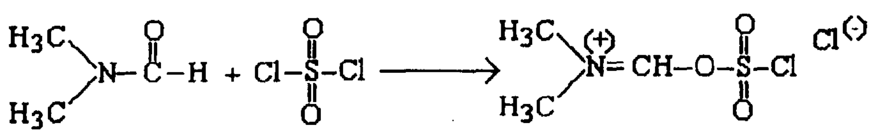

XV

**SCHEME B**

The DFCCS (XV) adduct thus obtained by the preferred process of the invention is found to be advantageous

in use in the process of manufacture of the reactive derivatives of formula I in accordance with the objective of the invention for the reasons given below.

i) Unlike the complex XIV used in the prior art, DFCCS (XV) used in the process of the present invention remains stable and does not get converted to the normal Vilsmeier's reagent. It has been observed that DFCCS (XV) of the present invention is apparently more stable than DFCS (XIV) described in USP 5 037 988. In particular it is found that the thus obtained DFCCS (XV) used in the process of the invention is distinct from thionyl chloride-DMF adduct i.e. dimethyl formiminium chloride chlorosulfite (XIV) known in the art. The melting point of the latter is 138°C - 140°C [Helv. Chim Acta, 62, 1655 (1959)] while that of the DFCCS adduct (XV) is 40°C - 41°C [Z. Chem 6(4), 148(1966)].

ii) The DFCCS (XV) used in the process of the invention can be prepared in any solvent such as benzene, toluene, acetonitrile or dichloromethane and also in the absence of solvents. This is advantageous and clearly distinct from the thionyl chloride-DMF adduct i.e. dimethyl formiminium chloride chlorosulfite (XIV) described in USP 5 037 988, which cannot be prepared in solvents such as chloroform or dichloromethane, since these solvents facilitate complete or partial conversion of adduct XIV to normal Vilsmeier's reagent.

iii) It has been found that sulfuryl chloride-DMF adduct(DFCCS) of formula XV is more stable than the DFCS adduct of formula XIV made from thionyl chloride and DMF. Thus, when DFCCS (XV) adduct was kept at ambient temperature for 16 hours and used for further complexation with compounds of formulae III, IV and V for synthesis of the activated ester required for the final acylation reaction, the drop in yield in the final antibiotic was about 26% (85% when used fresh and 59% after storage of DFCCS for 16 hours). Similarly when DFCS adduct was kept at ambient temperature for 16 hours and further processed for synthesis of the final antibiotic, the drop in yield was about 35% (80% when used fresh and 45% when used after 16 hours). Hence, DFCCS adduct (XV) obtained by the preferred process described above is having superior stability compared to DFCS adduct (XIV) and this is a great advantage for cost effective manufacture of cephalosporin antibiotics like cefotaxime, ceftriaxone, cefazolin and ceftazidime.

Thus, the use of DFCCS (XV) for synthesis of compound of formula Ia or Ib or Ic, provides practical, cost effective and safe method for manufacture of the desired cephalosporin antibiotics.

Also, in the present invention it was observed that when the preparation of DFCCS (XV) was carried out in the presence of 0.5 to 0.7 volume of dichloromethane as the solvent, DFCCS (XV) of improved quality was obtained.

Thus, in the present invention DFCCS (XV) was prepared by reacting equimolar quantities of N,N-dimethyl formamide and sulfuryl chloride in 0.5 volume of dichloromethane at 20°C to 25°C for 60 to 90 minutes.

The process of manufacture of the desired cephalosporin antibiotics of formula II such as cefotaxime (IIa) and ceftriaxone (IIb) according to the improved process of the invention basically involves the following:

a. the 2-(2-aminothiazol-4-yl)-2-(Z)-methoxyimino acetic acid (III) was activatived with DFCCS(XV) in dichloromethane at a temperature ranging from -30°C to -15°C to yield reactive form Ia.

b. The reactive derivative form Ia of step (a) was treated with silylated 7-amino cephalosporanic acid (XVIa) at a temperature ranging from -70°C to -30°C to yield the desired cephalosporins such as cefotaxime(IIa), ceftriaxone (IIb).

The process of the present invention for the preparation of cefotaxime and ceftriaxone described above may be represented as follows in scheme C:

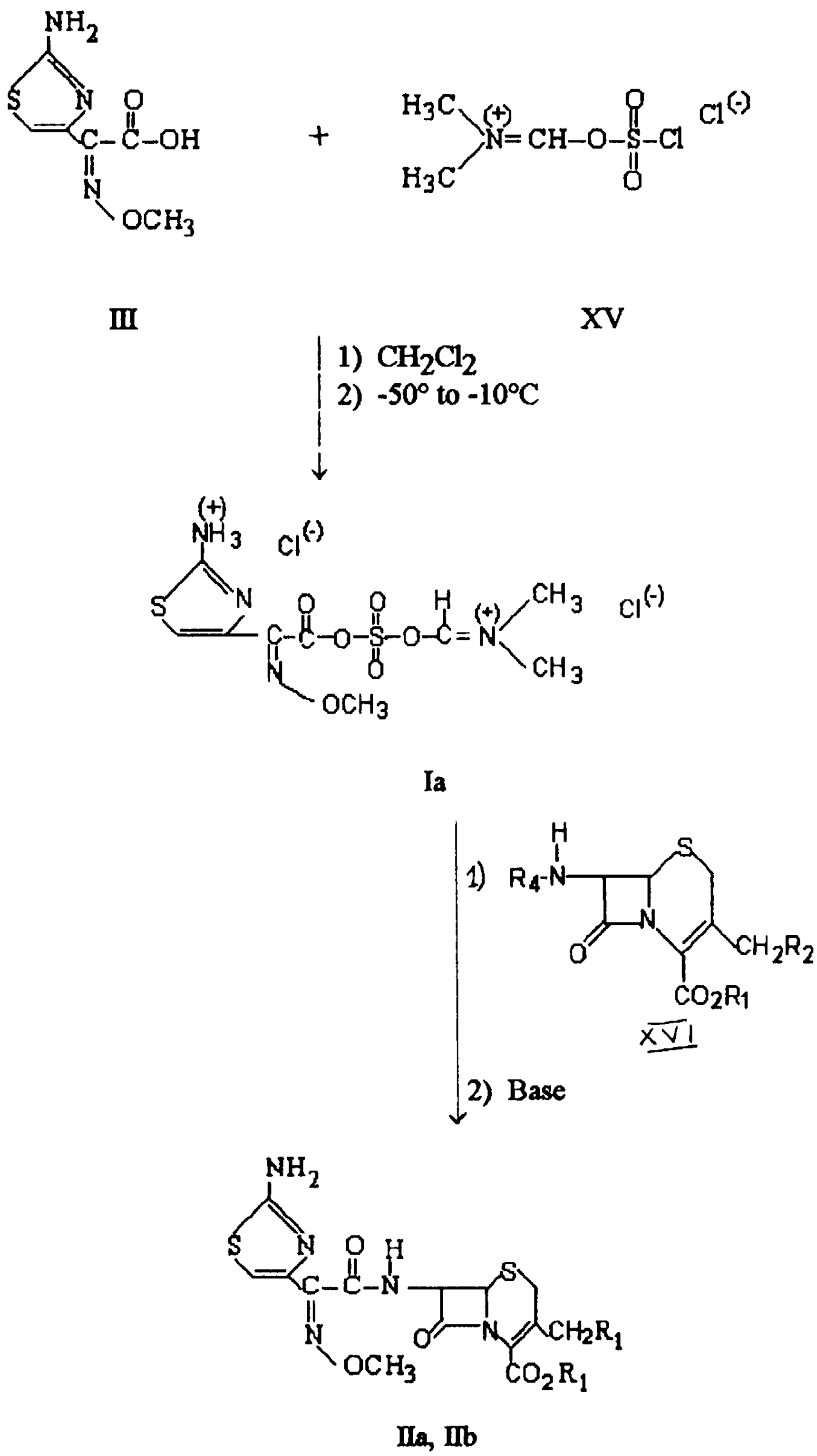


**SCHEME C**

Cefazolin (IIc) is prepared from silylated 7-amino-3-(2-methyl-1,3,4-thiadiazole-5-yl)thiomethyl-3-cephem-4-carboxylic acid and reactive derivative of 1H-tetrazol-1-acetic acid of formula Ib in a manner similar to that described for the preparation of cefotaxime (IIa), ceftriaxone (IIb).

The preparation of cefazolin (IIc) is illustrated in the following scheme D:

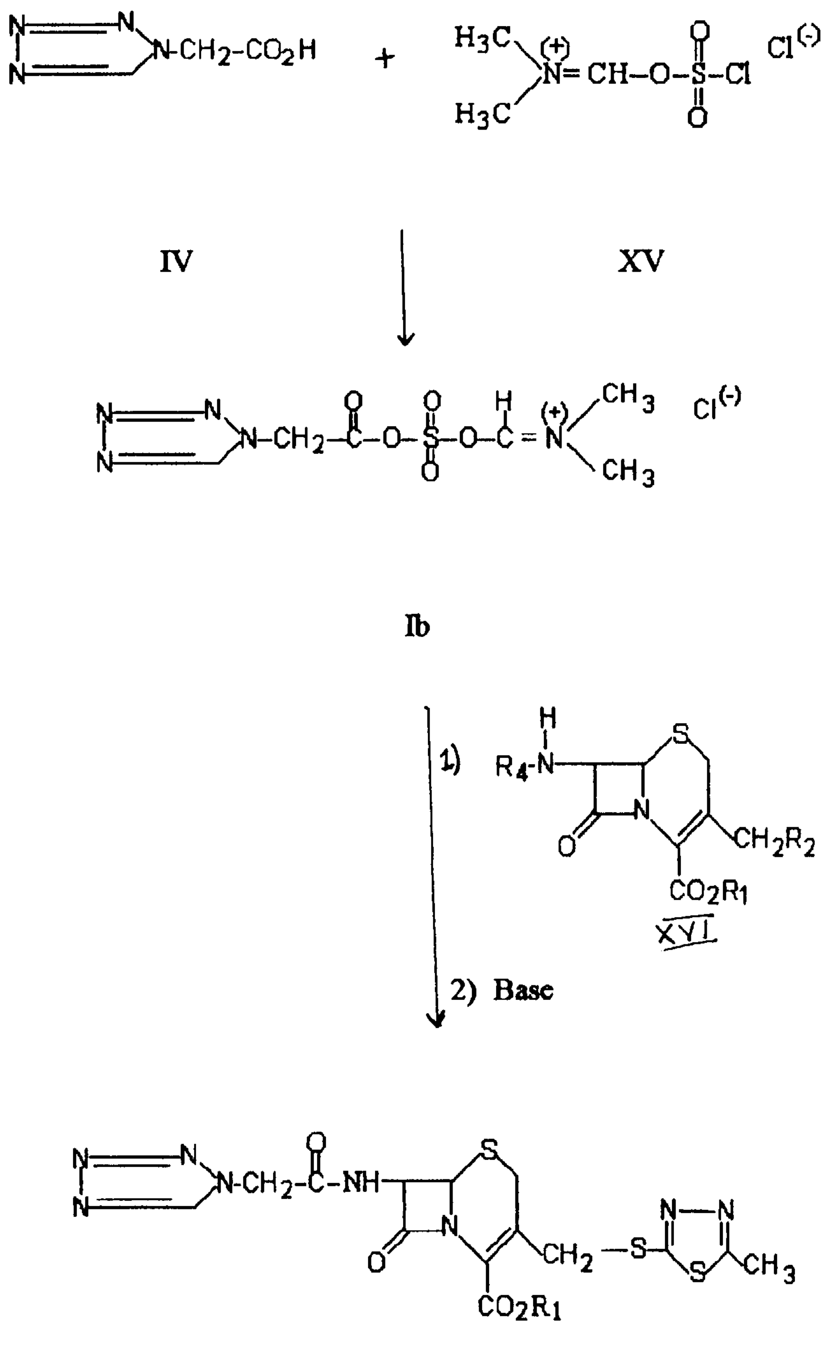


IV

XV

Ib

IIc

**SCHEME D**

The process of the present invention for the manufacture of ceftazidime (IId) is described in detail as follows:

In Step I 2-(2-aminothiazol-4-yl)-(Z)-2-[(1-tertbutoxycarbonyl-1-methylethoxy) imino]acetic acid (V) was activated with N,N-dimethylformiminium chloride chlorosulfate (XV) in dichloromethane at a temperature ranging from -20°C to -25°C. The temperature of the resulting reaction mixture is raised to -15°C ± 2°C to yield the reactive derivative of formula Ic.

In Step II 7-amino-3-(1-pyridiniummethyl)-3-cephem-4-carboxylic acid (VII) was silylated with a silylating agent such as N,O-bis(trimethyl silyl)acetamide in dichloromethane in the presence of an acid scavenging agent such as N,N-dimethylaniline at a temperature ranging from 18°C to 25°C.

In Step III the pre cooled reactive derivative of formula Ic is added to cooled silylated 7-amino-3-(1-pyridiniummethyl)-3-cephem-4-carboxylic acid(XVId) at a temperature ranging from -70° to -60°C to yield ceftazidime tertbutyl ester (XII) which was further converted to ceftazidime pentahydrate by procedure disclosed in the prior art.

Ceftazidime tertbutyl ester(XII) was first converted to ceftazidime dihydrochloride by following the procedure disclosed in USP 4 258 041 which in turn was converted to the stable pentahydrate form of ceftazidime(IId) by the procedure disclosed in USP 4 329 453.

The process of the present invention is illustrated in the following scheme E.

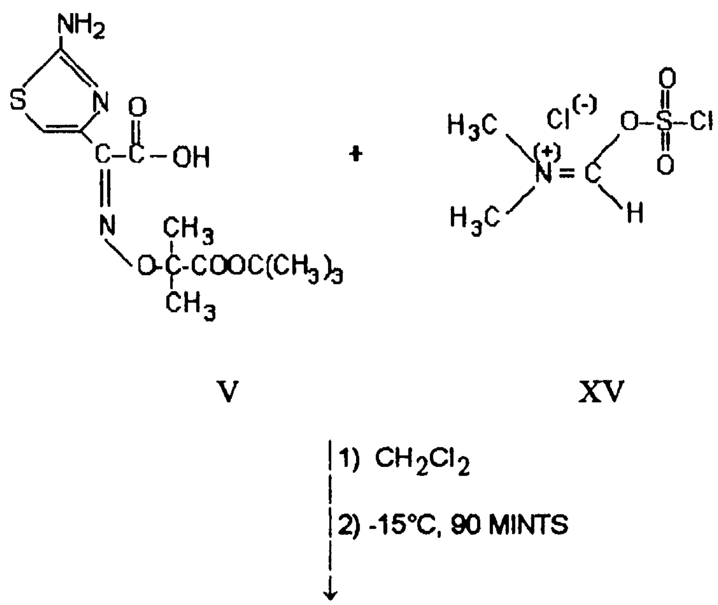


V

XV

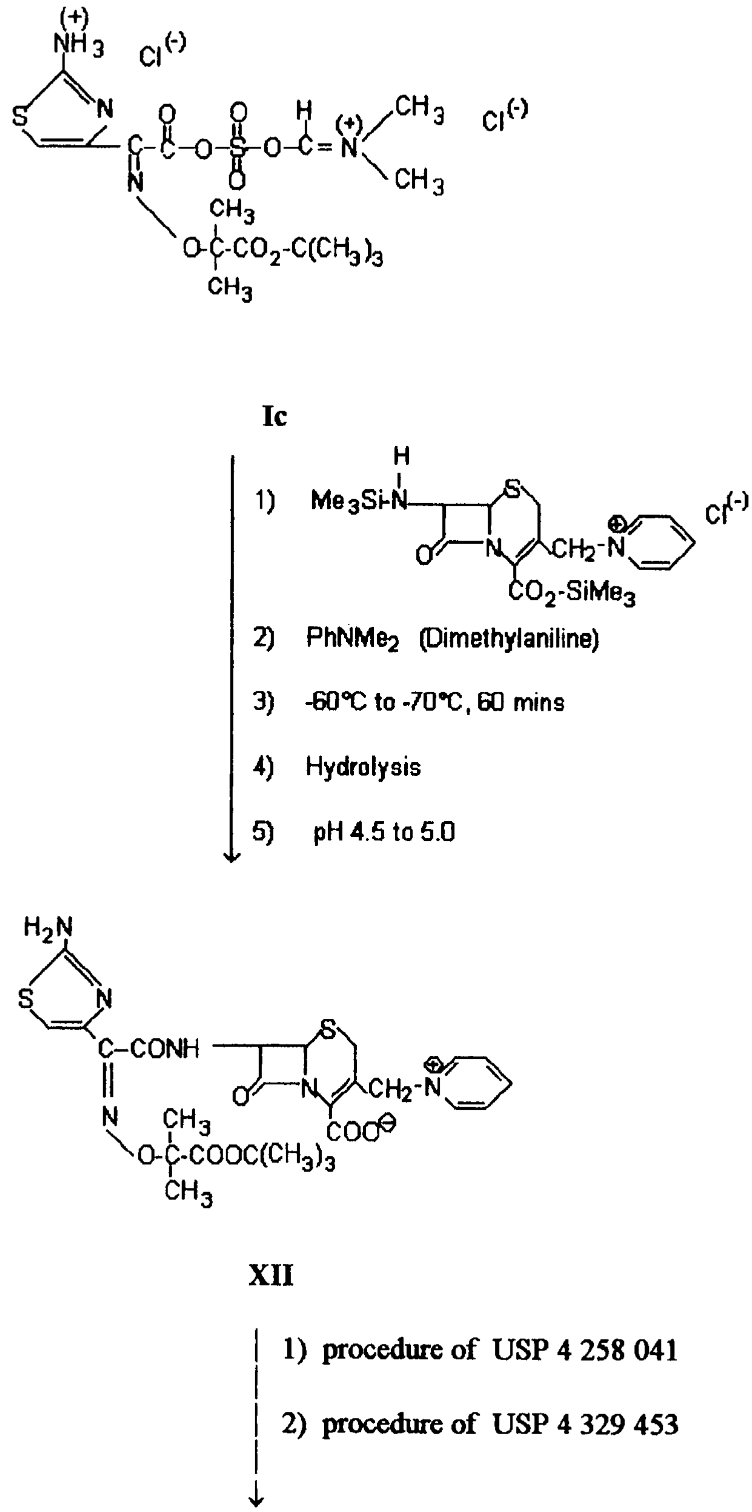


Ic

XII

IId

**CEFTAZIDIME PENTAHYDRATE**

**SCHEME E**

In the present invention the silylation is carried out by using conventional silylating agents such as N,O-bis (trimethylsilyl)acetamide, hexamethyl disilazane(HMDS), trimethylchlorosilane.

However, in the case of ceftazidime(IId) the preferred silylating agent is N,O-bis(trimethylsityl)acetamide and 4 moles of which is used per mole of the substrate.

In case of other cephalosporins such as cefotaxime(IIa), ceftriaxone(IIb) and cefazoline(IIc) alternatively the 7-ACA derivative is used inthe form of its quaternary salt with tetramethyl guanidine.

The acid scavenging agent used to capture the HCl released during silylation is selected from N,N-dimethylaniline, diethylamine, pyridine preferably N,N-dimethylaniline.

The solvent that is used during activation of addendum acids III, IV and V is selected from dichloroemethane, chloroform, benzene, toluene preferably dichloromethane.

The acylation reaction is carried out at a temperature ranging from -70°C to -30°C preferably -70°C to -55°C.

The process of the invention its objects and advantages would be further apparent from the non limiting examples illustrated hereunder:

## EXPERIMENTAL

### Example 1

Preparation of 7-[(2-(2-aminothiazol-4-yl)-2-syn-methoxyimino)acetamido] cephalosporanic acid (cefotaxime).

A) Activation of the 2-(2-aminothiazol-4-yl)-2-synmethoxyimino acetic acid.

11.9 g of sulfuryl chloride was added dropwise to 6.4 g of dimethylformamide at -20°C. The temperature was slowly raised to 0° C at which the solid adduct crystallised out. This was stirred vigorously for 1 hour and 50 ml of dichloromethane was added to the solid crystals. The temperature was raised to 15°C - 20° C and at this temperature the crystallised adduct melted and formed an immiscible layer with dichloromethane. The lower portion (N, N-Dimethyl forminium chloride chloro sulphate) was added to a pre cooled slurry of 16.02 g of 2-(2-aminothiazol-4-yl)-2-synmethoxyimino acetic acid in 150 ml of dichloromethane at -20°C to get a clear solution, which was kept for 1 hour at this temperature.

B) Preparation of 7-amino cephalosporanic acid solution.

20.0g of 7-aminocephalosporanic acid was taken in 150 ml of dichloromethane and 12.24g of hexamethyldisilazane was added to it, followed by refluxing for two hours. The clear solution obtained was cooled to 10°C and 12.1 ml of dimethylaniline was added to it, followed by cooling to -55°C.

C) Acylation

The reaction mixture (A) was cooled to -55°C and the reaction mixture (B) was added to it to get a clear solution. The temperature was maintained at -55°C for 10 minutes. The quantification of the condensed mass showed the formation of 82.5% of cefotaxime acid .

PMR (DMSO-$d_6$) δppm : 2.00(s), 3.3(q), 3.8(s), 4.9(q), 4.97(q), 5.60(2d), 6.70(s), 7.2(bs), 9.47(d)

D) Isolation of 7-[(2-(2-aminothiazol-4-yl)-2-syn-methoxyimino)acetamido] cephalosporanic acid.

200 ml of water was added to the above condensed mass and the temperature was brought to 25°C in 20 minutes. At this temperature, the pH of the hydrolysed mass was brought to 6.5 by using triethylamine. The aqueous layer was taken and 40 ml of 85% formic acid was added at 30°C. The product in the formic acid solvate form started precipitating after 10 minutes. This was cooled to 5°C and was stirred for one hour and then filtered to yield 29 g (87%) of the formic acid solvate with a purity of 92%.

UV max = 233.0nm (in water)

E) Conversion of formic acid solvate to isopropyl alcohol solvate of cefotaxime:

The above said formic acid solvate was taken in 175 ml of isopropyl alcohol and heated to 50°C for 4-5 hours. The isopropyl alcohol solvate thus formed was cooled to 5°C and then filtered to yield 26.97g (81%) of the isopropyl alcohol solvate with a purity of 92%.

MP = 202°-205°

a) IR : (main bands) in cm .

3420 (-$NH_2$ ), 3340 (-NH, -$NH_2$ ), 1760 (-C=O lactam),1730 (-C=O, carboxylic), 1650 (-C(=O)-NH), 1385 - 1355

(-O-CO-$CH_3$).

b) UV Characteristics :
UV max = 235.5nm (in water), E(1%,1 cm) = 376.00

c) Specific rotation :
[a] = +61.232 (C =1% aqueous solution)

F) Conversion of Isopropyl alcohol solvate of cefotaxime to cefotaxime sodium:
10.0g of the above solvate was taken in 60 ml of methanol and 11 ml of water and the mixture was cooled to +5 C. To this was slowly added 1.8 g of sodium acetate dissolved in 20 ml methanol to get a clear solution. To the above solution 400 ml of isopropyl alcohol was added within one hour to get cefotaxime sodium in the crystal form.

Example 2

Preparation of 7-[(2-(2-aminothiazol-4-yl)-2-syn-methoxyimino) acetamido] cephalosporanic acid (cefotaxime).

Same procedure as in example 1 was followed, but 400 ml of dichloromethane was used for the activation of 2-(2-amino thiazol-4-yl)-2-syn-methoxyimino acetic acid. Cefotaxime was prepared in 81.4% yield.

Example 3

Preparation of 7-[(2-(2-aminothiazol-4-yl)-2-syn-methoxyimino) acetamido] cephalosporanic acid (cefotaxime).

A) Activation of 2-(2-aminothiazol-4-yl)-2-syn-methoxyimino acetic acid:
Same procedure as in example 1 was followed.

B) Preparation of 7-aminocephalosporanic acid solution:
Same procedure as in example 1 was followed.

C) Acylation:
The reaction mixture (A) was cooled to -70°C and reaction mixture (B) was added to it to get a clear solution. The temperature of the condensed mass was maintained at -70°C for 10-15 minutes. The quantification of the condensed mass showed the formation of 75.5% of cefotaxime.

U) Isolation of 7-[(2-(2-aminothiazol-4-yl)-2-syn-methoxyimino) acetamido]cephalosporanic acid.
Same procedure as in example 1 was followed.

Example 4

Preparation of 7-[(2-(2-aminothiazol-4-yl)-2-syn-methoxyimino)acetamido] cephalosporanic acid (cefotaxime)

A) Activation of 2-(2-aminothiazol-4-yl)-2-syn-methoxyimino acetic acid.
19.8 g of sulfuryl chloride was added dropwise to 10.7g of dimethylformamide at -20°C. The temperature was slowly raised to 0°C at which white solid crystallises out. The reaction mixture was stirred vigorously for one hour. 60 ml of dichloromethane was added to the solid crystals and the temperature was brought to 15 -20°C. At this temperature, the solid crystals melted to form immiscible layer. The lower layer was added to a pre-cooled slurry of 15.08 g of 2-(2-aminothiazol -4-yl)-2-syn-methoxyimino acetic acid in 120 ml of dichloromethane at -20°C to get a clear solution which was kept for one hour at this temperature.

B) Preparation of 7-aminocephalosporanic acid solution:
Same procedure as in example 1 was followed.

C) Acylation:

The reaction mixture A was cooled to -55°C and the reaction mixture B was added to it to get a clear solution. The temperature was maintained at -50°C for 10 minutes. The quantification of the condensed mass showed only 36.3% of cefotaxime.

D) Isolation of 7-[(2-(2-aminothiazol-4-yl)-2-syn-methoxyimino)acetamido] cephalosporanic acid:
Same procedure as in example 1 was followed.

Example 5 :

Preparation of 7-[(2-(2-aminothiazol-4-yl)-2-syn-methoxyimino)acetamido] cephalosporanic acid (cefotaxime)

A) Activation of 2-(2-aminothiazol-4-yl)-2-syn-methoxyimino acetic acid:
10.9 g of sulfuryl chloride was added dropwise to 5.9 g of dimethylformamide at -20°C. The temperature was slowly increased to 0°C at which the solid adduct crystallised out. This was vigorously stirred for 1 hour at this temperature. To the solid crystals, 30 ml of dichloromethane was added and the temperature was raised to 15° -20°C. At this temperature the solid crystals melted and formed an immiscible layer with dichloromethane. The lower layer was added to pre-cooled slurry of 15.08 g of 2-(2-aminothiazol-4-yl)-2-syn-methoxyimino acetic acid in 120 ml of dichloro methane at -20°C to get a clear solution which was kept for one hour at the same temperature.

B) Preparation of 7-amino cephalosporanic acid solution :
Same procedure as in example 1 was followed.

C) Acylation :
The reaction mixture A was cooled to -55°C and reaction mixture B was added to it to get a clear solution. The temperature of the condensed mass was kept at -50°C for 10 minutes. The quantification of the condensed mass showed 60% of cefotaxime.

D) Isolation of 7-[(2-(2-aminothiazol-4-yl)-2-syn-methoxyimino)acetamido]cephalosporanic acid:
Same procedure as in example 1 was followed.

Example 6 :

Preparation of 7-[(2-(2-aminothiazol-4-yl)-2-syn-methoxyimino)acetamido]cephalosporanic acid (cefotaxime).

A) Activation of 2-(2-aminothiazol-4-yl)-2-syn-methoxyimino acetic acid.
Same procedure as in example 1 was followed.

B) Preparation of 7-aminocephalosporanic acid solution:
Same procedure as in example 1 was followed.

C) Acylation:
The reaction mixture A was cooled to -55 C and the reaction mixture B was added to it to get a clear solution. The temperature was maintained at -50 C for 30 minutes, instead of 10 minutes. The quantification of condensed mass showed 55% of cefotaxime.

D) Isolation of 7-[(2-(2-aminothiazol-4-yl)-2-syn-methoxyimino)acetamido] cephalosporanic acid :
Same procedure as in example 1 was followed.

Example 7 :

Preparation of 7-[(2-(2-aminothiazol-4-yl)-2-syn-methoxyimino)acetamido] cephalosporanic acid (Cefotaxime).

A) Activation of 2-(2-aminothiazol-4-yl)-2-syn-methoxyimino acetic acid:
Same procedure as in example 1 was followed.

B) Preparation of 7-aminocephalosporanic acid solution:
20.0 g of 7-aminocephalosporanic acid was taken in 150 ml of dichloromethane and 12.24 g of hexamethyldisilazane was added to it followed by refluxing for 2 hours. The clear solution obtained was cooled to 10 C and 9.4 g of acetamide was added to it followed by cooling to -55°C.

C) Acylation :
Same procedure as in example 1 was followed. Quantification of the condensed mass showed 60% of cefotaxime.

D) Isolation of 7-[(2-(2-aminothiazol-4-yl)-2-synmethoxyimino)acetamido] cephalosporanic acid:
Same procedure as in example 1 was followed

Example 8 :

Preparation of 7-[(2-(2-aminothiazol-4-yl)-2-synmethoxyimino)acetamido] cephalosporanic acid (Cefotaxime).

A) Activation of 2-(2-aminothiazol-4-yl)-2-synmethoxyimino acetic acid:
11.9 g of sulfuryl chloride was added dropwise to 6.4 g of dimethylformamide at -2 C. The temperature was slowly raised to 0° C at which the solid adduct crystallised out. This was stirred vigorously for 1 hour and 50 ml of dichloromethane was added to the solid crystals. The temperature was raised to 15°C-20 °C and at this temperature the crystallised adduct melted and formed an immiscible layer with dichloromethane. The lower portion (N,N-Dimethyl forminium chloride chloro sulphate) was added to a pre cooled slurry of 16.25 g of 2-(2-aminothiazol-4-yl)-2-synmethoxyimino acetic acid in 150 ml of dichloromethane at -20°C to get a clear solution, which was kept for 1 hour at this temperature.

B) Preparation of 7-amino cephalosporanic acid solution.
20.0g of 7-aminocephalosporanic acid was taken in 120 ml of dichloromethane and 10.65g of hexamethyl disilazane and 0.1 g imidazole was added to it. Start refluxing for 4 hours under nitrogen atmosphere.(After 30 minutes to refluxing, clear solution was obtained) Cooled to 10° C and 12.1 ml dimethylaniline was added followed by cooling to 55° C.

C) Acylation
The reaction mixture (A) was cooled to -55°C and the reaction mixture (B) was added to it to get a clear solution. The temperature was maintained at -55°C for 10 minutes. The quantification of the condensed mass showed the formation of 90 to 95% of cefotaxime acid depending on the potency of the starting product (7-ACA).

D) Isolation of 7-[(2-(2-aminothiazol-4-yl)-2-syn-methoxyimino)acetamido] cephalosporanic acid.
80 ml of water was added to the above condensed mass and the temperature was brought to 25°C in 20 minutes. At this temperature, the pH of the hydrolysed mass was brought to 6.5 by using triethylamine. The aqueous layer was separated. Organic layer was extracted with 20 X 2 ml DM water. Combined aqueous layer and extraction was taken and 40 ml of 85% formic acid was added at 30°C. The product in the formic acid solvate form started precipitating after 10 minutes. This was cooled to 0°C - 5°C and was stirred for one hour and then filtered to yield 30 g (87%) of the formic acid solvate with a purity of 92%.

E) Conversion of formic acid solvate to isopropyl alcohol solvate of cefotaxime:
Same procedure as in example 1 was followed.

Example 9 :

Preparation of 7-(1-(1H)-tetrazolylacetamido)-3-[2- (5-methyl-1,3,4-thiadiazolyl)thiomethyl]-3-cephem-4-carboxylic acid (Cefazolin).

A) Activation of 1H-tetrazol-1-acetic acid:

9.41 g of sulfuryl chloride was added dropwise to 5.09 g of dimethylformamide at -20°C. The temperature was slowly raised to 0°C at which the solid adduct crystallised out. To this 50 ml of dichloromethane was added and the solid crystals melted to form an immiscible layer. The lower layer was added to 8.9 g of 1H-tetrazol-1-acetic acid in 120 ml of dichloromethane at -20°C. The temperature was raised to 0° C which resulted in the formation of a turbid reaction mass. This reaction mass was kept at 0°C for one hour.

B) Preparation of 7-amino-3-[2-(5-methyl-1,3,4-thiadiazolyl)thiomethyl]-3-cephem-4-carboxylic acid solution:

20.0 g of 7-amino-3-[2-(5-methyl-1,3,4-thiadiazolyl)thiomethyl]-3-cephem-4-carboxylic acid was taken in 120 ml dichloromethane and to this 9.39 g of hexamethyl disilazane was added and refluxed for 2 hours to get a clear solution. After 2 hours the clear solution was cooled to 10°C and 12.6 g dimethylaniline was added to it and the reaction mixture was further cooled to -40°C.

C) Acylation:

The reaction mixture A was cooled to -40°C, to which reaction mixture B was added and the temperature of the condensed mass was maintained at -25°C for 10-15 minutes. The quantification of condensed mass showed 53% of cefazolin.

D) Isolation of 7-(1-(1H)-tetrazolylacetamido)-3-[2-(5-methyl-1,3,4-thiadiazolyl)thiomethyl]-3-cephem-4-carboxylic acid.

To the above condensed mass, 100 ml of water was added and the pH was brought to 6 by using triethylamine. The layer was separated and the pH of the aqueous layer was adjusted to 1.5 to yield white crystals of cefazolin. This was filtered and washed with water followed by methanol.

a) IR : (Main bands) in cm
3280 (-NH), 3140, 3075 (N=N, -C=N- tetrazole ring), 2620, 2580 (-OH, bonded, -COOH), 1770 (C=O lactam), 1715 (C=O acid), 1670 (C=O amide-I), 1555 (C=O amide-II).

b) UV Characteristics :
UV max = 272.5 nm (in water), E(1%, 1cm) = 290.76

c) Specific rotation :
$[\alpha]_D^{20}$ = -19.6° (C=1% aqueous solution)

Example 10 :

Preparation of 7-(1-(1H)-tetrazolylacetamido)-3-[2-(5-methyl -1,3,4-thiadiazolyl)thiomethyl]-3-cephem-4-carboxylic acid(cefazolin)

A) Activation of 1H-tetrazol-1-acetic acid.

Same procedure as in example 9 was followed. But, 140 ml of dichloromethane was used for activation.

B) Preparation of 7-amino-3-[2-(5-methyl-1,3,4-thiadiazolyl)thiomethyl]-3-cephem-4-carboxylic acid solution:

20.0 g of 7-amino-3-(2-methyl-1,3,4-thiadiazol-5-yl) thiomethyl-3-cephem-4-carboxylic acid was taken in 140 ml dichloromethane and hexamethyldisilazane was added and refluxed for two hours to get a clear solution. This solution was cooled to 10°C and 12.6 g of dimethylaniline was added. The resulting mixture was cooled to -40°C.

C) Acylation :

The reaction mixture A was cooled to -40°C to which reaction mixture B was added and the temperature of

the resulting condensed mass was maintained at -25°C for 10-15 minutes. The quantification of the condensed mass showed 61% of cefazolin.

D) Isolation of 7-(1-(1H)-tetrazolylacetamido)-3-[2-(5-methyl-1,3,4-thiadiazolyl)thiomethyl]-3-cephem-4-carboxylic acid.

Same procedure as in example 9 was followed.

Example 11 :

Preparation of 7-{[(2-aminothiazol-4-yl)(methoxyimino) acetyl)amino]-8-oxo-3-{[(1,2,5,6-tetrahydro-2-methyl-5,6-dioxo-1,2,4-triazin-3-yl)thiolmethyl}-5-thia-1-azabicyclo(4.2.0) oct-2-ene-2-carboxylic acid (ceftriaxone) :

A) Activation of 2-(2-aminothiazol-4-yl)-2-syn-methoxyimino acetic acid :

9.06 g of sulfuryl chloride was slowly added to 4.91 g of dimethylformamide at -25°C. The temperature was slowly raised to 0°C in 60-90 minutes. At this temperature white solid i.e. dimethyl forminium chloride chlorosulfate (DFCCS) crystallised out 50 ml of dichloromethane was added to the solid crystals and stirred vigorously for 15-20 minutes when the solid crystals melted to form an immiscible white crystalline coloured oil which was decanted in the lower portion. 11.37 g of 2-(2-aminothiazo]-4-yl)-2-syn-methoxyimino acetic acid in 120 ml of dichloromethane was cooled to -30°C and to this the above immiscible layer was added in 20-30 minutes. The temperature was slowly raised to -10°C in one hour to get a clear solution.

B) 7-amino-3-desacetoxy-3-[(2,5-dihydro-6-hydroxy-3-methyl -5-oxo-as-triazin-3-yl)thio] cephalosporanic acid solution.

20.0 g of 7-amino-3-desacetoxy-3-[(2,5-dihydro-6 -hydroxy-3-methyl-5-oxo-as-triazin-3-yl)thio)]cephalosporanic acid in 160 ml of dichloromethane. To the resulting reaction mixture 40 ml of N, O-bis-silyl acetamide was added and stirred for 8 hours at 30°C to get a clear solution, cooled to -20°C and 8.35 ml dimethylaniline was added to it and the reaction mixture was finally cooled to -50°C.

C) Acylation

The reaction mixture A was cooled to -30°C and added to the reaction mixture B. The temperature was maintained at -40°C for 20-30 minutes.

Thick precipitation occurred which became thin during stirring, HPLC showed the formation of 70% of ceftriaxone.

PMR [DMSO-$d_6$) δ ppm : 3.3(d), 3.59(s), 3.9(s), 4.2(d), 5.1(d), 5.72(d), 6.8 (s), 9.7(d).

D) Isolation :

After 30 minutes of the reaction, 200 ml of water was added at -40°C and stirred for 30 minutes at 20-25°C. The pH of the reaction mass was brought to 6.5 by adding triethylamine. The aqueous layer was separated and was treated with 5.0 g of activated carbon and 2.0 g of sodium bisulfite for 30 minutes, filtered and washed with 40 ml water. The pH is then adjusted to 3.2 by 1:1 HCl, seeded and the resulting mixture was stirred for 30 minutes at 25°-30°C. Finally the pH is readjusted to 2.6 and the reaction mixture was stirred for 20 minutes and then further stirred for two hours at 0°C to -5°C. The product was filtered, washed with chilled isopropyl alcohol to give 21 g (70%) of ceftriaxone, Assay = 92.88% - 95%

a) IR Characteristics
Main band = 1780 cm (β lactam)

b) UV Characteristics
UV max = 241.5nm(in water), E(1%, 1cm) = 481.46

c) Specific rotation : $[\alpha]_D^{20}$ = -153.88°.
(C = 1% aqueous solution)

Example: 12

Preparation of 7-[[(2-aminothiazol-4-yl)-2-syn-methoxyimino] acetamido]-8-oxo-3-[(1,2,5,6-tetrahydro-2-methyl-5,6-dioxo-1,2,4-triazin-3-yl)thiomethyl] cephalosporanic acid (ceftriaxone)

A) Activation of 2-(2-aminothiazol-4-yl)-2-syn-methoxyimino acetic acid.
Same procedure as in example 11 was followed.

B) Quaternary salt of 7-amino-3-desacetoxy-3-(2,5-dihydro-6-hydroxy-3-methyl-5-oxo-as-triazin-3-yl)thio) cephalosporin acid.
20.0 g of 7-amino-3-desacetoxy-3-(2,5-dihydro-6-hydroxy-3-methyl-5-oxo-as-triazin-3-yl)thio)cephalosporanic acid in 120 ml of dichloromethane was cooled to -15°C and 12.3 g of tetramethyl guanidine (TMG) was added to it. The temperature was slowly raised to - 20°C to -25°C in one hour. The reaction mixture was stirred for one hour at 25°C to get a clear solution. This solution was further cooled to -30°C to -35°C and 8.35 ml dimethylaniline was added to it and the reaction mixture was finally cooled to -40°C.

C) Acylation :
Same procedure as in example 11 was followed. HPLC showed formation of 75% of ceftriaxone:

D) Isolation.
Same procedure as in example 11 was followed.

Example 13 :

Preparation of 7-[(2-aminothiazol-4-yl)(methoxyimino) acetamido]-8-oxo-3-[[(1,2,5,6-tetrahydro-2-methyl-5,6-dioxo-1,2,4-triazin-3-yl)thio]methyl] cephalosporanic acid (ceftriaxone)

A) Activation of 2-(2-aminothiazol-4-yl)-2-syn-methoxyimino acetic acid.
Same as procedure as in example 11 was followed.

B) 7-amino-3-desacetoxy-3-[(2,5-dihydro-6-hydroxy-3-methyl -5-oxo-as-triazin-3-yl)thiolcephalosporanic acid solution.
20.0 g of 7-amino-3-desacetoxy-3-(2,5-dihydro-6 -hydroxy-2-methyl-5-oxo-as-triazin-3-yl)thio)cephalosporanic acid in 200 ml of dichloromethane was taken and 19.05 g of trimethylsilyl chloride(TMCS) was added to it, followed by refluxing for 3 hours to give clear solution, cooled to -20°C and 8.53 ml dimethylamine was added to it. The reaction mixture was finally cooled to -50°C.

C) Acylation:
Same procedure as in example 11 was followed HPLC showed conversion to 70% of ceftriaxone.

Example: 14

Preparation of 7-[[(2-aminothiazol-4-yl)-2-syn-methoxyimino] acetamido]-8-oxo-3-[(1,2,5,6-tetrahydro-2-methyl-5,6-dioxo-1,2,4-triazin-3-yl)thiomethyl] cephalosporanic acid (ceftriaxone)

A) Activation of 2-(2-aminothiazol-4-yl)-2-syn-methoxyimino acetic acid.
8.72 g of sulfuryl chloride was slowly added to 4.7 g of dimethylformamide at -25°C. The temperature was slowly raised to 0 C in 60-90 minutes. At this temperature white solid i.e. dimethyl forminium chloride chlorosulfate (DFCCS) crystallises out at 0° C. 50 ml of dichloromethane was added to the solid crystals and stirred vigorously for 15-20 minutes when the solid crystals melt to form a colourless immiscible oil which was decanted and taken separately. 11.37 g of 2-(2-aminothiazol-4-yl)-2-syn-methoxyimino acetic acid in 120 ml of dichloromethane and was cooled to -30°C and to this the above immiscible oily layer was added in 20-30 minutes. The temperature was

slowly raised to -10°C in one hour to get a clear solution and was kept at this temperature for 30 minutes.

B) 7-amino-3-desacetoxy-3-[(2,5-dihydro-6-hydroxy-3-methyl -5-oxo-as-triazin-3-yl)thio] cephalosporanic acid solution.

20.0 g of dry 7-amino-3-desacetoxy-3-[(2,5-dihydro-6 -hydroxy-3-methyl-5-oxo-as-triazin-3-yl)thio)]cephalosporanic acid in 120 ml of dichloromethane to which 13.05 g of hexamethyldisilazane, 2.5 g of trimethyl chlorosilane and 0.5 g of imidazole were added and refluxed for 5 to 6 hours to get a hazy reaction mixture. This was cooled to 10°C and 8.3 ml of dimethylaniline was added and was further cooled to -50°C.

C) Acylation :

The reaction mixture (A) was cooled to -50° C and reaction mixture (B) was added to it to get a clear solution and the temperature of the resulting reaction mixture was maintained at -45°C to -50°C for 30 minutes. HPLC monitoring showed the formation of 86% ceftriaxone.

D) Isolation:

After 30 minutes, 200 ml of water and 50 ml of triethylamine was added to get pH 6.5 - 7.0. The layer as separated and the aqueous layer was taken and isopropyl alcohol (20ml) and ethyl acetate(40 ml) was added to it and stirred for 10 minutes at 20°C to 25°C. The pH of this aqueous layer was adjusted to 2.5 by using formic acid to get white crystals. This was further cooled to 0° to 5°C and maintained for one hour. After one hour the product was filtered and washed with 50 ml of water followed by 50m of chilled isopropanol to give 25.8q (86.5%) ceftriaxone acid having a 98% purity.

Example 15:

Preparation of (6R,7R)-7-[[(2-amino-4-thiazolyl)-2-(1-carboxy-1-methyl ethoxy)imino]acetamido]-3-(1-pyridiniummethyl)-3-cephem-4-carboxylate pentahydrate (ceftazidime pentahydrate)

I. Preparation of (6R,7R)-7-amino-3-(1-pyridiniummethyl)-3-cephem-4-carboxylic acid iodide monohydrate.

Iodine (117 g, 915 mmol) was taken in dichloromethane (750 ml) at room temperature and the temperature was further raised to 36°C. Hexamethyl disilazane (HMDS)(80g, 496 mmole) was added to it at 35°C over 30 hrs and the reaction mixture was refluxed for 4 to 5 hours till the colour disappeared. Pyridine (90g, 1168 mmole) was added to the reaction mixture in 60 minutes at 30°C followed by addition of 7-aminocephalosporanic acid (50 g, 183 mmol) at 35°C - 40°C. The reaction was monitored by HPLC. The reaction mixture was cooled to 10°C and isopropyl alcohol (220 ml) and water (30 ml) was added to it. The resulting reaction mixture was further cooled to 0°C to 2°C for 2 hours, filtered and the cake was washed with a mixture of isopropanol (80 ml) and water(5 ml), followed by acetone (100 ml).

The resulting product was taken in methanol (200 ml) at 10°C and dissolved by adding 17% HCl, stirred for 60 minutes. The pH of the resulting solution was adjusted to 2.8 to 3.0 by addition of triethylamine. The resulting solution was cooled to 0°C - 5°C for 60 minutes, filtered with a mixture of methanol and acetone (50:50) followed by chilled acetone (50 ml) and dried under vacuum at 35°C for 2 hours to obtain 62 g (90%) of the product

Water Content (KF) : 3 - 4%

IR (KBr) $cm^{-1}$ : 3300, 1790, 1600, 1480, 1400, 1140.

II. Preparation of (6R,7R)-7-amino-(1-pyridiniummethyl)-3-cephem-4-carboxylic acid hydrochloride monohydrate.

The compound of Step I (10g) was dissolved in 0.1N HCl (50 ml) at 20°C. The resulting product was precipitated by adding isopropanol (250 ml) at 20°C, filtered, washed with isopropanol (10ml) and dried under vacuum for 2 hours at 40°C to obtain 7.5 g of the product.

Water Content (KF): 4 - 5%

IR : (KBr) $cm^{-1}$ : 3300, 1780, 1600.

III. Preparation of (6R,7R)-7-[(2-(2-amino-4-thiazolyl)-2-[(1-tertbutoxy carbonyl-1-methylethoxy)imino]acetamido-3-(1-pyridiniummethyl)-3-cephem- 4-carboxylate (ceftazidime tert butyl ester).

A) Silylation of (6R,7R)-7-amino-3-(1-pyridiniummethyl)-3-cephem-4-carboxylic acid hydrochloride monohydrate.

(6R,7R)-7-amino-3-(1-pyridiniummethyl)-3-cephem-4-carboxylic acid hydrochloride monohydrate(10g,

26 mmoles) was taken in dichloro methane (100 ml) and then 40 ml of dichloromethane was distilled out. After cooling the reaction mixture to 25°C, N,O-bis(trimethyl silyl)acetamide (21.2g, 104 mmole) was added to it and agitated for 4 hours to get a clear solution. The reaction mixture was cooled to -70°C and N,N-dimethylaniline(4.2g, 34 mmol) was added to it.

B) Preparation of dimethyl formiminium chloride chlorosulfate (DFCCS)

Sulfuryl chloride (4.761 g, 34 mmol) was taken in dichloromethane (5 ml) and the reaction mixture was cooled to -25°C. N,N-dimethylformamide (2.54 g, 34 mmole) was added to the reaction mixture slowly in 30 minutes and the temperature was raised to 22°C and agitated for 90 minutes at the same temperature at which the oily layer separates out. Further, an additional 10 ml of dichloromethane was added to it and agitated for another 10 minutes. The oily layer containing DFCCS was separated out.

C) Activation of (Z)-2-(2-aminothiazol-4-yl)-2-[(1-tertbutoxy-carbonyl-1-methylethoxy)imino] acetic acid.

(Z)-2-(2-aminothiazol-4-yl)-2-[(1-tertbutoxycarbonyl-1-methylethoxy) imino]acetic acid (8.84 g, 26 mmol) was taken in dichloromethane (50 ml) and the resulting reaction mixture was cooled to -25°C and the above oily layer containing DFCCS was added to it in 30 minutes. The temperature of the reaction mixture was raised to -10°C to get a clear solution and agitated for 90 minutes and then cooled to -60°C.

D) Acylation:

The pre-cooled reactive derivative of step (c) was added to pre-cooled silylated 7-amino-3-(1-pyridiniummethyl)-3-cephem-4-carboxylic acid and the temperature of the reaction mass was kept at -70°C for 90 minutes. The reaction was monitored by HPLC which showed 70% formation of the product. To the reaction mixture 40 ml of water was added and the aqueous layer was separated. The pH of the aqueous layer was adjusted to 5.2 by adding 8% sodium hydroxide solution. The reaction mixture was cooled to 5°C, stirred for 3-4 hours, and the resulting product obtained was filtered, washed with chilled water (20 ml) and dried in vacuum to obtain 9.5 g (64%) of ceftazidime tert butyl ester.

MP : 138°-140°C.

IR(KBr) $cm^{-1}$ : 3400, 1780, 1720, 1610, 1530.

UV (0.1 N $H_2SO_4$): λmax = 256 nm

NMR [DMSO-$d_6$], δ(ppm): 1.4(S,9H), 1.55(S,6H), 3.6(AB,2H), 5.3(d, 1H),5.6(S,2H), 5,92(d, 1H), 6.98(S, 1H), 8.1 - 9.1(5H, pyridinium)

Specific Rotation : $[\alpha]_D^{20}$ = -31° (C=1% in DMSO)

IV) Preparation of ceftazidime dihydrochloride.

Ceftazidime tert butyl ester (10g, 16.6 mmol) was dissolved in a mixture of trifluroacetic acid (40 ml) and water (40 ml) at 15°C and the resulting solution was stirred for 6 hours at the same temperature followed by addition of water (3 ml) and acetone (180 ml) at 30°C. The reaction mixture was heated to 45°C and a mixture of HCl and acetone (40:40) was added to it over a period of 90 minutes to get a thick precipitate. The reaction mixture was cooled to 0°C and stirred for 2 hours. The resulting product was washed with chilled acetone, filtered, dried in vacuum to obtain 8.0 g of ceftazidime dihydrochloride.

MP : 165°-168°C.

UV (0.1N $H_2SO_4$): λmax = 256 nm

IR (KBr) in $cm^{-1}$ : 3500 - 2900, 1780, 1720

NMR [DMSO-$d_6$] δ(ppm) :1.45(S,6H), 5.26(d,1H), 5.84(d,1H), 6.92(S,1H), 8 - 9.1 (pyridinium)

Specific Rotation: $[\alpha]_D{}^{20}$ = - 14.2° ( C = 0.95% in pH 6 buffer )

Chlorine content = 9.8 - 10%

Water Content (KF) = 4.5 - 5%

V) Preparation of ceftazidime pentahydrate.

A. Crude Ceftazidime Pentahydrate.

Ceftazidime dihydrochloride (8g, 12.9 mmol) was dissolved in water (35 ml). The resulting solution was cooled to 15°C and agitated for 10 minutes and the pH of the solution was adjusted to 3-3.2 by Amberlite LA-2 solution. Further the pH of the resulting solution was readjusted to 4.2 by using Amberlite LA-2 solution and agitated for 18 hours at 0°C to 5°C. The product obtained was filtered, washed with chilled ethyl acetate (12 X 2 ml) followed by chilled acetone (12 ml), and dried under vacuum at 40°C to obtain 6.75 g of crude ceftazidime pentahydrate.

Specific Rotation : $[\alpha]_D{}^{20}$ = + 124.2°(C= 1% in I)MSO)

B. Purification of crude ceftazidime pentahydrate

The crude ceftazidime pentahydrate (5g, 9 mmol) was dissolved in water (10 ml) at 10°C. The solution was cooled to 5°C and the pH was adjusted to 5.8 to 6 by sodium hydroxide solution to get a clear solution. To the clear solution 0.1 g carbon was added and filtered. The carbon bed was washed with water (2 X 2 ml). The filtrate and the washings were combined and the pH of the solution was adjusted to 4.4 at 10°C by using 85 % formic acid. The solution was seeded, agitated for 2 hours at 10°C. The reaction mass was cooled to 0°C and further agitated for another 15 hours. The pH of the reaction mixture was adjusted to 4.2 by using formic acid and further agitated for another 2 hours at 0°C. The resulting product was filtered, washed with chilled water (10 ml) followed by chilled acetone (10 ml)and dried in vacuum to obtain 3.8 g of pure ceftazidime pentahydrate.

MP = 135°-138°C

UV (0.1N $H_2SO_4$): λmax = 256.4 nm

IR (KBr) in $cm^{-1}$: 3500 - 3000, 1760, 1720, 1540, 1490, 1160, 680.

NMR [250MHz,DMSO-$d_6$-$D_2O$] δ(ppm): 7.25(S,2H),6.6(S, 1H), 5.7(d,2H), 5.65(d,1H), 5.2(d,1H),5.05(d, 1H) 3.5(d,1H), 3.05(d,1H), 1.38(S,6H), 9.3,8.6, 8.15(pyridinium)

Specific Rotation : $[\alpha]_D^{20}$ = +25° (C=1% DMSO)

Example 16

I. Preparation of (6R,7R)-3-acetoxymethyl-7-[2-(2-amino-thiazol-4-yl)-(Z)-2-[(1-tertbutoxycarbonyl-1-methylethoxy)imino]acetamido] (-3-cephem-4-carboxylic acid.

A. Silylation of 7-amino cephalosporanic acid (7-ACA)

7-ACA(10g, 36 mmol) was taken in dichloromethane (60 ml) and hexamethyldisilazane (5.35g, 33 mmol) was added to it. The reaction mixture was refluxed with stirring for 4 hours to get a clear solution. After cooling the resulting reaction mixture to -40°C, N,N-dimethylaniline (5.25 g, 42 mmol) was added to it and further cooled to -60°C.

B. Preparation of dimethyl formiminium chloride chlorosulfate (DFCCS).

Sulfuryl chloride (6.4 g, 47 mmol) was taken in dichloromethane (60 ml) and the reaction mixture was cooled to -25°C. N,N-dimethylformamide (3.5 g, 47 mmol) was added to the reaction mixture slowly in 30 minutes. The temperature of the reaction mixture was slowly raised to 22°C at which a very viscous oily layer separates out which was stirred for 90 minutes at 22°C. Further an additional 10 ml of dichloromethane was added to it and stirred for another 15 minutes. The oily layer containing DFCCS was separated out.

C. Activation of 2-(2-aminothiazol-4-yl)-(Z)-2-[(1-tertbutoxycarbonyl methylethoxy)imino]acetic acid.

2-(2-aminothiazol-4-yl)-(Z)-2-[(1-tertbutoxycarbonyl-1-methylethoxy) imino]acetic acid(12g, 36 mmol) was taken in dichloromethane (60 ml) and the resulting reaction mixture was cooled to -24°C. The above oily layer of DFCCS was added to it in 30 minutes at -25°C. The temperature of the reaction mixture was raised to -10°C and stirred for 90 minutes to get a clear solution. This reaction mixture was cooled to -60°C.

D. Acylation:

The pre-cooled reactive derivative of step C was added to pre - cooled silylated 7-ACA at -60°C. The temperature of the reaction mixture was raised to -50°C to -45°C. The reaction when monitored by HPLC showed 90% formation of the product.

E. Hydrolysis:

To the reaction mass of step D, demineralised water (40 ml) and trimethylamine (25 ml) was added and the temperature was raised to 20°C. The resulting reaction mixture was stirred for 30 minutes at the same temperature and the pH of the reaction mixture was maintained at 6.8 to 7. The organic and the aqueous layers were separated. The aqueous layer was extracted with dichloromethane (20 X 2 ml). The organic layer was then combined and dichloromethane was removed by vacuum distillation when a sticky mass was obtained. Water (300 ml) was added to the sticky mass and the pH of the reaction mass was adjusted to 2.8 to 3.0 by cone. HCl. The resulting reaction mixture was cooled to 0°C and stirred for 3 - 4 hours. The product obtained was filtered washed with chilled diisopropyl ether(20 ml), dried in vacuum for 2 hours to obtain 16 g (75 - 80%) of the title compound having purity of 98% .

MP = 152°C - 160°C

IR (KBr) in $cm^{-1}$ : 3450, 3350, 1760, 1735, 1710

NMR [DMSO-$d_6$] δ(ppm): 1.38(S, 9H), 1.45(S, 6H), 2.1(S, 3H), 4.6(d,1H), 5.0(d, 1H), 5.2(d, 1H), 5.8(d, 1H), 6.75 (S, 1H), 7.35(S, 2H), 9.4(d, 1H).

II. Preparation of (6R,7R)-7-[(Z)-2-amino-thiazol-4-yl)-2-[(2-carboxy methylethoxy)imino]acetamido-3-acetoxymethyl-3-cephem-4-carboxylic acid.

The compound of step I (5 g) was treated with trifluoroacetic acid (20 ml) and water (2 ml), and agitated for 3 - 4 hours at 15°C. Alter 4 hours 95% of the hydrolysed product was obtained as monitored by HPLC. The reaction mass was added to diisopropyl ether (250 ml) when trifluoroacetic acid salt of the title compound was obtained. Diisopropyl ether was decanted and the white solid obtained was dissolved in water (10 ml). The pH of the resulting solution was adjusted to 7.0 by 12% ammonia solution. The pH was readjusted to 2.8 by 85% formic acid. The reaction mixture was cooled to 5"C under stirring and the resulting product was filtered, washed with chilled water (10 ml) to obtain 3.5 g of the title compound.
MP = > 270°C

$$\text{UV } \lambda\text{max(pH 6.0 buffer)} : 236 \text{ nm } (E^{1\%}_{1cm}\ 250),$$
$$\lambda\text{inf } 255 \text{ nm } (E^{1\%}_{1cm}\ 238),\ 296 \text{ nm}(E^{1\%}_{1cm}\ 103)$$
20

Specific Rotation : $[\alpha]^{20}_{D}$ = +20° (C - 1% DMSO)

III. Preparation of ceftazidime Pentahydrate

Sodium iodide (7.129 g, 47 mmol) was dissolved in water (3 ml) at 60°C. To the resulting solution, pyridine (3.69 g, 45 mmol) was added followed by the compound of step II (2.9 g, 4 mmol). The reaction mixture was agitated for 1 hour at 80°C and 44% of the product was formed as observed by HPLC. The reaction mixture was cooled and diluted with water (25 ml). The pH of the resulting reaction mixture was adjusted to 6 by using 2N sodium hydroxide. The aqueous layer was extracted with ethyl acetate (2 X 20 ml). A few drops of methyl isobutyl kctone was added to it. The pH of the solution was further adjusted to 1 by cone, HCl. the separated solid was filtered and the pH of the collected filtrate was slowly adjusted to 3.8 to 4.0 by using Amberlit LA-2 in 30 minutes and stirred for 15 hours at 0°C to 5°C. The product obtained was filtered, washed with small amount of ethyl acetate and acetone, and dried under vacuum to obtain 0.23 g of the title compound .

MP: 135°-138°C

UV (0.1N $H_2SO_4$): λmax = 256.4 nm

IR(KBr) in $cm^{-1}$ : 3500-3000, 1760, 1720, 1540, 1490, 1160, 680

NMR [25MHz,DMSO-$d_6$-$D_2O$] δ(ppm) :7.25(S, 2H), 6.6(S,1H), 5.7(d,2H),5.65(d, 1H), 5.2(d, 1H), 5.05(d,1H) 3.5(d, 1H), 3.05(d, 1H), 1.38(S,6H),9.3,8.6, 8.15(pyridinium)

Specific Rotation : $[\alpha]_D{}^{20}$ = +25° (C=1% DMSO)

## Claims

1. The compound of formula I

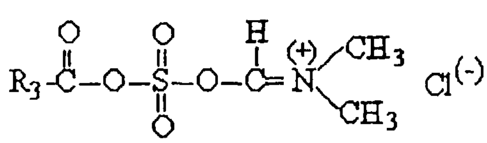

I

wherein

R3 = 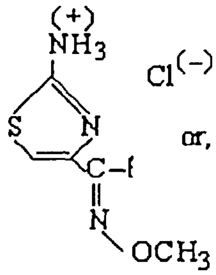 or, 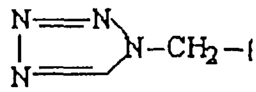 or, 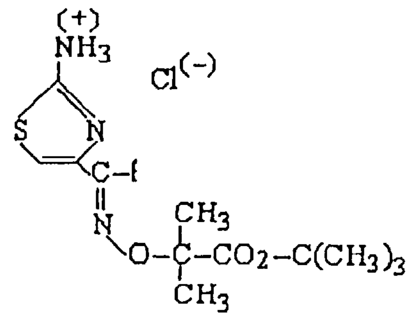

2. A process for the production of compound of formula I comprising reacting dimethyl formiminium chloride chloro sulphate (DFCCS) of formula XV,

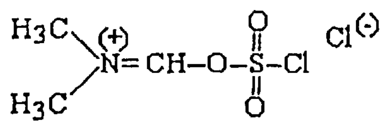

XV

with 2-(2-aminothiazol-4-yl)-(Z)-2-methoxyimino acetic acid or 1H-tetrazole-1-acetic acid or 2-(2-aminothiazol-4-yl)-(Z)-2-[(1-tertbutoxycarbonyl-1-methylethoxy) imino] acetic acid in dichloromethane, chloroform, benzene or toluene at a temperature ranging from -30°C to -15°C.

3. A process according to claim 2 wherein the temperature is about -20°C.

4. A process according to claim 2 or claim 3 wherein said DFCCS of formula XV is N,N-dimethyl formiminium chloride chlorosulphate obtained by reacting dimethyl formamide (DMF) with sulfuryl chloride, and said compound of formula I is a reactive derivative of formula Ia,

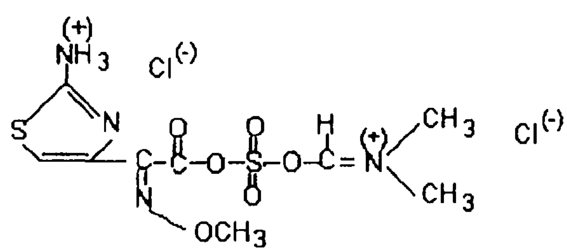

Ia

obtained by reacting said 2-(2-aminothiazol-4-yl)-(Z)-2-methoxyimino acetic acid with said DFCCS of formula XV

or

reactive derivative of formula Ib,

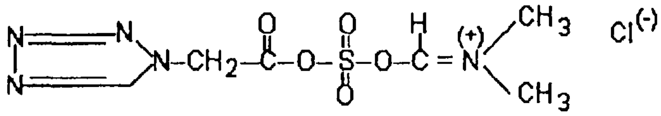

Ib

obtained by reacting said 1H-tetrazol-1-acetic acid with said DFCCS of formula XV

or

a reactive derivative of formula Ic

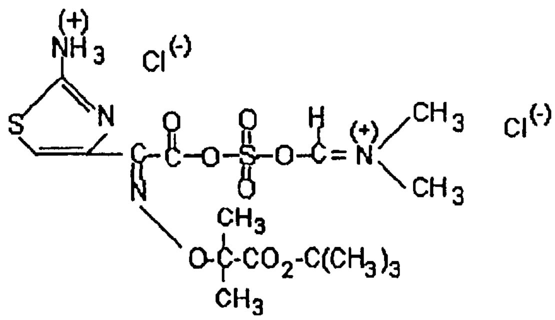

Ic

obtained by reacting said 2-(2-aminothiazol-4-yl)-(Z)-2-[(1-tertbutoxy carbonyl-1-methylethoxy)imino]acetic acid with said DFCCS of formula XV.

**5.** A process as claimed in claim 4 wherein the molar ratio of said DFCCS(XV) with respect to said 2-(2-aminothiazol-4-yl)-(Z)-2-methoxyimino acetic acid/said 1H-tetrazol-1-acetic acid is 1.1 to 1.2.

**6.** A process according to claim 5 wherein the molar ratio of said DFCCS (XV) with respect to said 2-(2-aminothiazol-4-yl)-(Z)-2 methoxyimino acetic acid/said 1H-tetrazol-1-acetic acid is 1.17.

**7.** A process according to claim 4 wherein the molar ratio of DFCCS(XV) to said 2-(2-aminothiazol-4-yl)(Z)-2-[(1-terbutoxycarbonyl-1-methylethoxy)imino] acetic acid (V) is 1.0 to 1.3.

**8.** A process for the preparation of cephalosporin antibiotics of formula II,

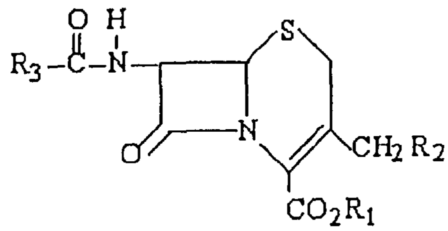

II

wherein R1 = H, carboxylic protecting group

IIa : $R_2$ = acetoxy , $R_3$ = 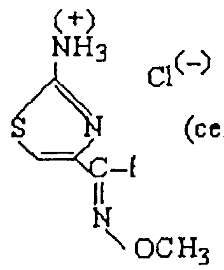 (cefotaxime)

IIb : R = 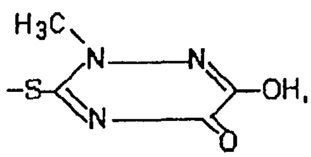 , $R_3$ = 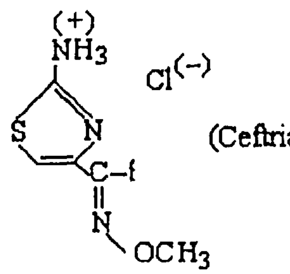 (Ceftriaxone)

which comprises reacting silylated 7-amino cephalosporanic acid derivatives of formula XVI

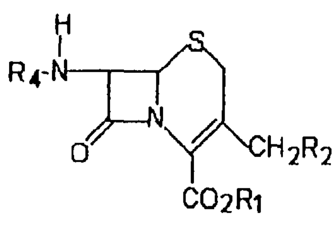

XVI

in which R1, R2 is as defined in said formula II; R4 = silyl group or hydrogen; with a reactive derivative of formula Ia in dichloromethane, chloroform, benzene or toluene at temperature ranging from -70°C to -30°C.

**9.** A process for the preparation of cephalosporin antibiotics of formula II

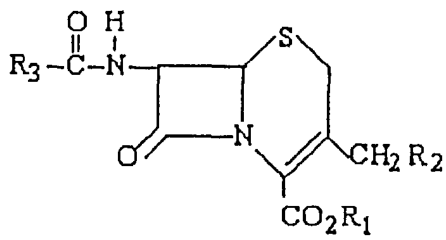

II

wherein R1 = H, carboxylic protecting group

IIc : $R_2$ = 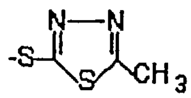, $R_3$ = 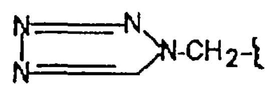 (Cefazolin)

which comprises reacting silylated 7-amino cephalosporanic acid derivatives of formula XVI,

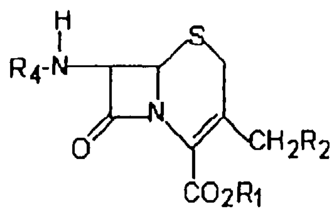

XVI

in which R1, R2 is as defined in said formula II; R4 = silyl group or hydrogen, with a reactive derivative of formula Ib in dichloromethane, chloroform, benzene or toluene at temperature ranging from -70°C to -30°C.

**10.** A process according to any one of claims 8 or 9 wherein the temperature ranges from -70° to - 65°.

**11.** A process for the preparation of ceftazidime of formula IId

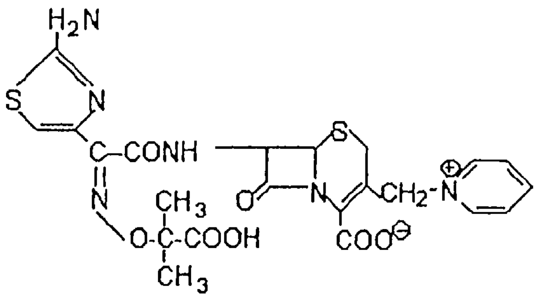

IId

which comprises:

a. silylating 7-amino-3-(1-pyridmiummethyl)-3-cephem-4-carboxylic acid of formula VII,

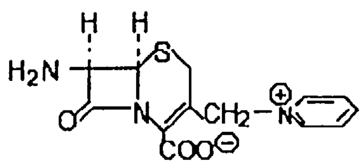

VII

with a silylating agent in the presence of an acid scavenging agent at a temperature ranging from 18°C to 25°C to provide silylated 7-amino-3-(1-pyridiniummethyl)-3-cephem-4-carboxylic acid of formula XVId

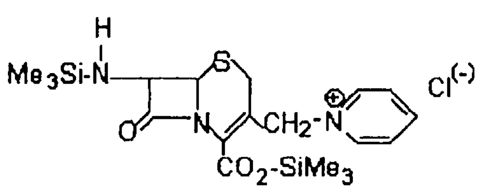

XVId

b. activating 2(2-aminothiazol-4-yl)-(Z)-2-[(1-tertbutoxycarbonyl-1-methylethoxy)imino]acetic acid(V)

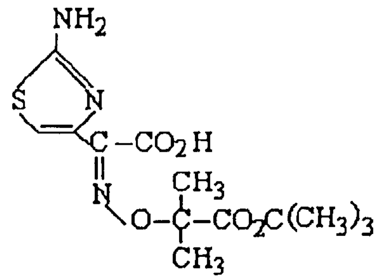

V

with N,N-dimethylformiminium chloride chlorosulfate(DFCCS) XV

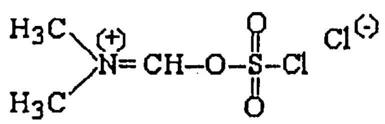

XV

in dichloromethane at a temperature ranging from -20°C to -25°C to provide reactive derivative of formula Ic;

c) acylating the silylated 7-amino-3-(1-pyridiniummethyl)-3-cephem-4-carboxylic acid (XVId) of step (a) with the reactive derivative of formula IC of step (b) at a temperature ranging from -70 C to -60 C to obtain ceftazidime tertbutyl ester (XII),

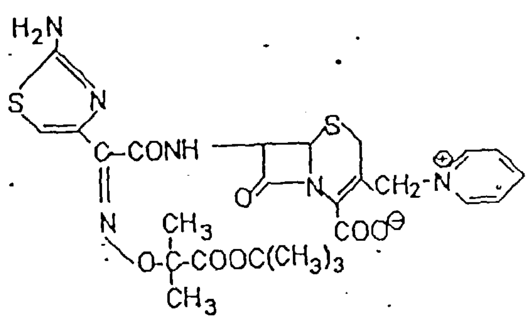

XII

which is converted to ceftazidime pentahydrate IId via ceftazidime dihydrochloride.

**12.** A process according to claim 11 wherein the silylated 7-amino-3-(1-pyridiniummethyl)-3-cephem-4-carboxylic acid (XVId) is reacted with said reactive derivative of formula IC in the presence of an acid scavenging agent.

**13.** A process according to claim 11 or 12 wherein the said acid scavenging agent is selected from N,N-dimethylaniline,

diethylamine, pyridine.

**14.** A process according to any one of claims 11 to 13 wherein the silylating agent is selected from N, O-bis(trimethylsilyl)acetamide, hexamethyldisilazane, trimethyl chlorosilane preferably N, O-bis(trimethylsilyl) acetamide.

**15.** A process according to any one of claims 11 to 14 wherein the molar ratio of DFCCS (XV) to (Z)-2-(2-aminothiazol-4-yl)-2- [(1-tertbutoxycarbonyl-1-methylethoxy) imino] acetic acid (V) is 1.0 to 1.3.

**16.** A process according to any one of claims 8 to 15 wherein the molar ratio of said reactive derivative of formula 1A or 1B or 1C with respect to said silylated 7-ACA is 1.1 to 2.

**17.** A process according to claim 16 wherein the molar ratio of said reaction derivative of formula 1A or 1B or 1C with respect to said silylated 7-ACA is 1.2.

**18.** A process for the preparation of DFCCS of formula XV comprising reacting dimethyl formamide (DMF) with sulfuryl chloride.

**19.** A process according to claim 18 wherein said DFCCS of formula XV is prepared by reacting equimolar amount of sulfuryl chloride and dimethyl formamide.

**20.** A process according to claim 18 or 19 wherein said N-N-dimethylformiminium chloride chlorosulphate (DFCCS) of formula XV used is obtained by reacting said N,N-dimethylformamide (DMF) and said sulfuryl chloride in the presence of 0.5 to 0.7 volume of dichloromethane as the solvent.

## Patentansprüche

**1.** Verbindung der Formel I:

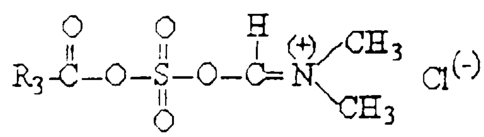

I

worin

$R_3$ = 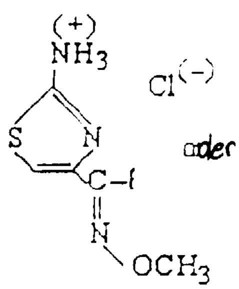 oder 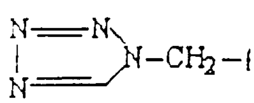 oder 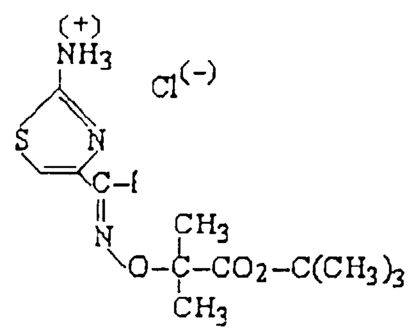

ist.

**2.** Verfahren zur Herstellung einer Verbindung der Formel I, umfassend das Umsetzen von Dimethylformiminiumchlorid-chlorsulfat (DFCCS) der Formel XV

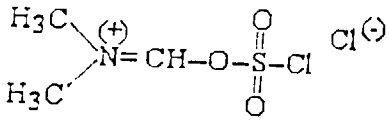

XV

mit 2-(2-Aminothiazol-4-yl)-(Z)-2-methoxyiminoessigsäure oder 1H-Tetrazol-1-essigsäure oder 2-(2-Aminothiazol-4-yl)-(Z)-2-[(1-tert-butoxycarbonyl-1-methylethoxy)imino]essigsäure in Dichlormethan, Chloroform, Benzol oder Toluol bei einer Temperatur im Bereich von -30°C bis -15°C.

**3.** Verfahren nach Anspruch 2, worin die Temperatur etwa -20°C beträgt.

**4.** Verfahren nach Anspruch 2 oder 3, worin das DFCCS der Formel XV N,N-Dimethylformiminiumchlorid-chlorsulfat ist, das durch Umsetzen von Dimethylformamid (DMF) mit Sulfurylchlorid erhalten wird, und die Verbindung der Formel I ein reaktives Derivat der Formel Ia

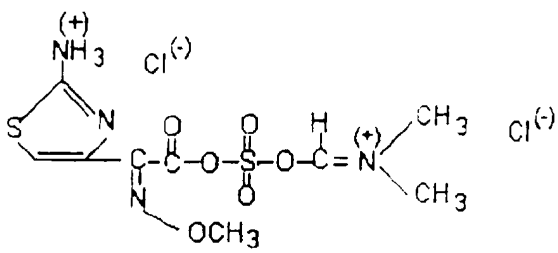

Ia

ist, das durch Umsetzen der 2-(2-Aminothiazol-4-yl)-(Z)-2-methoxyiminoessigsäure mit dem DFCCS der Formel XV erhalten wird, oder
ein reaktives Derivat der Formel Ib

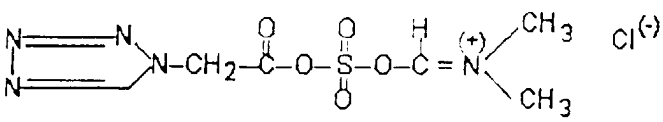

Ib

ist, das durch Umsetzen der 1H-Tetrazol-1-essigsäure mit dem DFCCS der Formel XV erhalten wird,
oder ein reaktives Derivat der Formel Ic

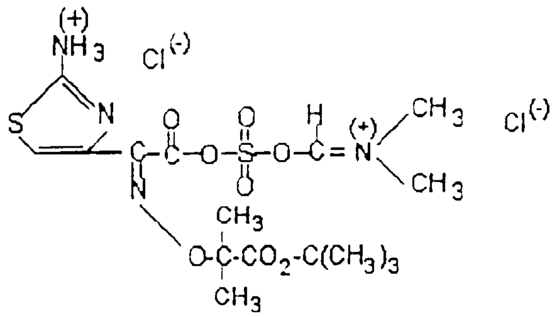

Ic

ist, das durch Umsetzen der 2-(2-Aminothiazol-4-yl)-(Z)-2-[(1-tert-butoxycarbonyl-1-methylethoxy)imino]essigsäure mit dem DFCCS der Formel XV erhalten wird.

**5.** Verfahren nach Anspruch 4, worin das Molverhältnis zwischen DFCCS (XV) und 2-(2-Aminothiazol-4-yl)-(Z)-2-methoxyiminoessigsäure/1H-Tetrazol-1-essigsäure 1,1 bis 1,2 beträgt.

**6.** Verfahren nach Anspruch 5, worin das Molverhältnis zwischen DFCCS (XV) und 2-(2-Aminothiazol-4-yl)-(Z)-2-methoxyiminoessigsäure / 1H-Tetrazol-1-essigsäure 1,17 beträgt.

**7.** Verfahren nach Anspruch 4, worin das Molverhältnis zwischen DFCCS (XV) und 2-(2-Aminothiazol-4-yl)-(Z)-2-[(1-tert-butoxycarbonyl-1-methylethoxy)imino]essigsäure (V) 1,0 bis 1,3 beträgt.

**8.** Verfahren zur Herstellung von Cephalosporin-Antibiotika der Formel II

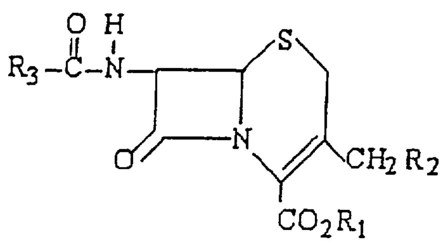

II

worin R1 = H, Carboxylschutzgruppe

IIa: $R_2$ = Acetoxy, R3 = 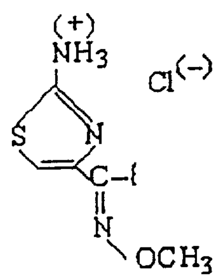 (Cefotaxim)

IIb: R = 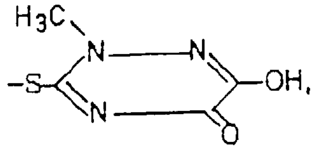 $R_3$ = 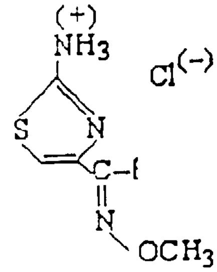 (Ceftriaxon)

sind, welches das Umsetzen von silylierten 7-Aminocephalosporansäure-Derivaten der Formel XVI

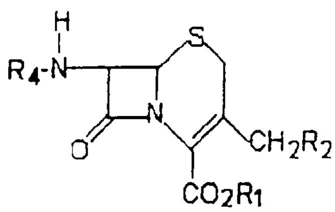

XVI

worin R1 und R2 wie in Formel II definiert sind; und R4 = eine Silylgruppe oder Wasserstoff ist;
mit einem reaktiven Derivat der Formel Ia in Dichlormethan, Chloroform, Benzol oder Toluol bei einer Temperatur im Bereich von -70°C bis -30°C umfasst.

**9.** Verfahren zur Herstellung von Cephalosporin-Antibiotika der Formel II

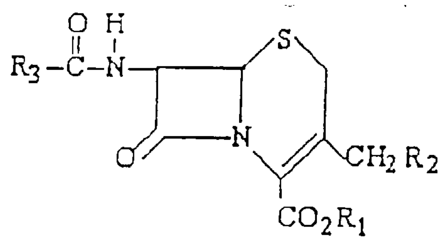

II

worin R1 = H, Carboxylschutzgruppe

IIc: $R_2$ = 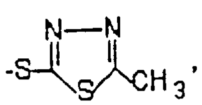 $R_3$ = 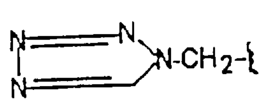 (Cefazolin)

sind, welches das Umsetzen von silylierten 7-Aminocephalosporansäure-Derivaten der Formel XVI,

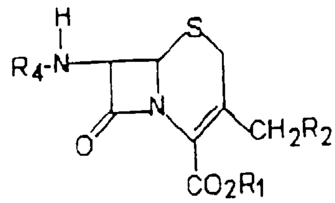

XVI

worin R1 und R2 wie in Formel II definiert sind; und R4 = eine Silylgruppe oder Wasserstoff ist; mit einem reaktiven Derivat der Formel Ib in Dichlormethan, Chloroform, Benzol oder Toluol bei einer Temperatur im Bereich von -70 °C bis -30 °C umfasst.

**10.** Verfahren nach einem der Ansprüche 8 oder 9, worin die Temperatur im Bereich von -70° bis -65° liegt.

**11.** Verfahren zur Herstellung von Ceftazidim der Formel IId

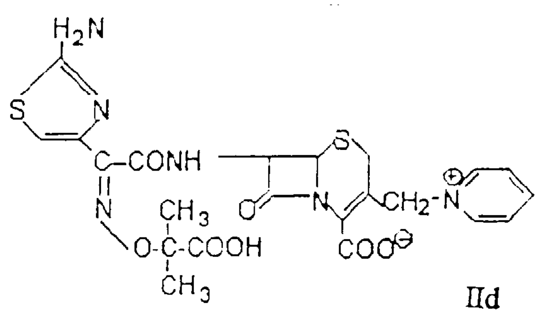

IId

welches Folgendes umfasst:

a) das Silylieren von 7-Amino-3-(1-pyridiniummethyl)-3-cephem-4-carbonsäure der Formel VII

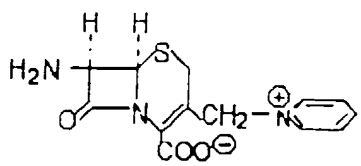

VII

mit einem Silylierungsmittel in Gegenwart eines Säurefängers bei einer Temperatur im Bereich von 18 °C bis 25 °C, um silylierte 7-Amino-3-(1-pyridiniummethyl)-3-cephem-4-carbonsäure der Formel XVId

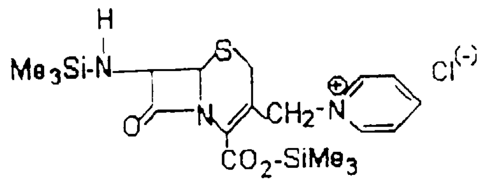

XVId

bereitzustellen;

b) das Aktivieren von 2-(2-Aminothiazol-4-yl)-(Z)-2-[(1-tert-butoxycarbonyl-1-methylethoxy)imino]essigsäure (V)

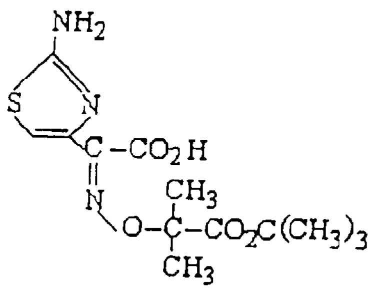

V

mit N,N-Dimethylformiminiumchlorid-chlorsulfat (DFCCS) XV

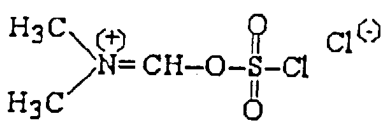

XV

in Dichlormethan bei einer Temperatur im Bereich von -20 °C bis -25 °C, um das reaktive Derivat der Formel Ic bereitzustellen;

c) das Acylieren der silylierten 7-Amino-3-(1-pyridiniummethyl)-3-cephem-4-carbonsäure (XVId) aus Schritt a) mit dem reaktiven Derivat der Formel Ic aus Schritt b) bei einer Temperatur im Bereich von -70 °C bis -60 °C, um Ceftazidim-tert-butylester (XII)

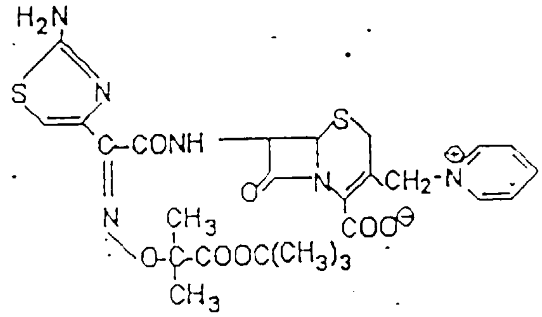

XII

zu erhalten, der über Ceftazidim-dihydrochlorid in Ceftazidim-pentahydrat IId übergeführt wird.

**12.** Verfahren nach Anspruch 11, worin die silyliert 7-Amino-3-(1-pyridiniummethyl)-3-cephem-4-carbonsäure (XVId) mit dem reaktiven Derivat der Formel Ic in Gegenwart eines Säuretängers umgesetzt wird.

**13.** Verfahren nach Anspruch 11 oder 12, worin der Säurefänger aus N,N-Dimethylanilin, Diethylamin und Pyridin ausgewählt ist.

**14.** Verfahren nach einem der Ansprüche 11 bis 13, worin das Silylierungsmittel aus N,O-Bis(trimethylsilyl]acetamid, Hexamethyldisilazan und Trimethylchlorsilan ausgewählt ist und vorzugsweise N,O-Bis(trimethylsilyl)acetamid ist.

**15.** Verfahren nach einem der Ansprüche 11 bis 14, worin das Molverhältnis zwischen DFCCS (XV) und (Z)-2-(2-Aminothiazol-4-yl)-2-[(1-tert-butoxycarbonyl-1-methylethoxy)imino]essigsäure (V) 1,0 bis 1,3 beträgt.

**16.** Verfahren nach einem der Ansprüche 8 bis 15, worin das Molverhältnis zwischen dem reaktiven Derivat der Formel Ia oder Ib oder Ic und der silylierten 7-ACA 1,1 bis 2 beträgt.

**17.** Verfahren nach Anspruch 16, worin das Molverhältnis des reaktiven Derivats der Formel Ia oder Ib oder Ic und der silylierten 7-ACA 1,2 beträgt.

**18.** Verfahren Zur Herstellung von DFCCS der Formel XV, welches das Umsetzen von Dimethylformamid (DMF) mit Sulfurylchlorid umfasst.

**19.** Verfahren nach Anspruch 18, worin das DFCCS der Formel XV durch Umsetzen äquimolarer Mengen an Sulfurylchlorid und Dimethylformamid hergestellt wird.

**20.** Verfahren nach Anspruch 18 oder 19, worin das eingesetzte N-N-Dimethylformiminiumchlorid-chlorsulfat (DFCCS) der Formel XV durch Umsetzen des N,N-Dimethylformamids (DMF) und des Sulfurylchlorids in Gegenwart von 0,5 bis 0,7 Volumina Dichlormethan als Lösungsmittel erhalten wird.

**Revendications**

**1.** Composé de la formule I

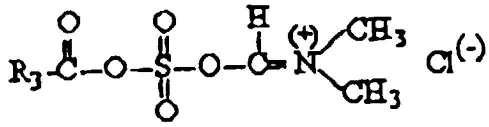

I

où

$R_3$ =

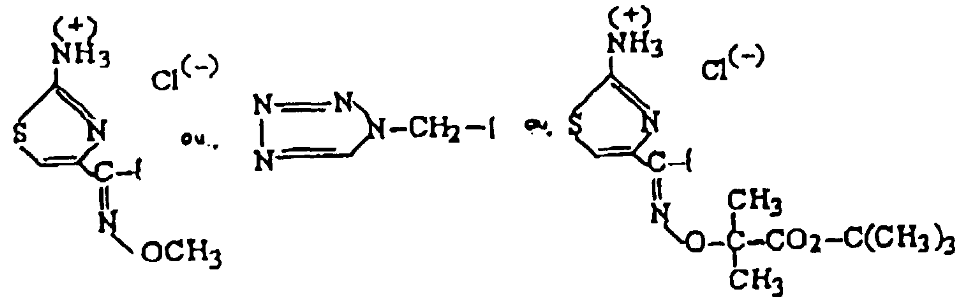

ou

ou

2. Procédé pour la production du composé de formule 1 comprenant la réaction du chlorure de diméthyl formiminium chlorosulfaté (DFCCS) de formule XV,

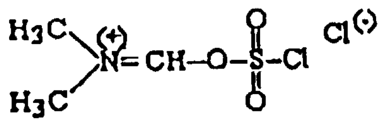

XV

avec de l'acide 2-(2-aminothiazol-4-yl)-(Z)-2-méthoxy-imino acétique ou de l'acide 1H-tétrazole-1-acétique ou de l'acide 2-(2-aminothiazol-4-yl)(Z)-2-[(1-tertbutoxycarbonyl-1-méthyléthoxy)imino] acétique dans le dichlorométhane, le chloroform, le benzène ou le toluène à une température comprise entre -30°C et -15°C.

3. Procédé selon la revendication 2 où la température est d'environ -20°C.

4. Procédé selon la revendication 2 ou la revendication 3 où ledit DFCCS de formule XV est le chlorure de N,N-diméthyl foromiminium chlorosulfaté obtenu par réaction de diméthyl formamide (DMF) avec du chlorure de sulfuryle et ledit composé de formule 1 est un dérivé réactif de formule 1a,

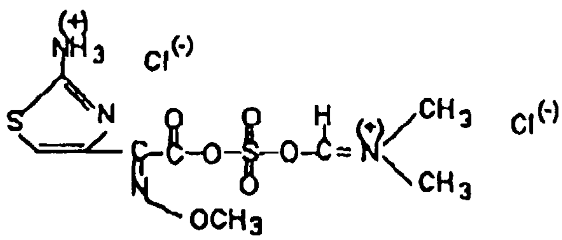

Ia

obtenu par réaction dudit acide 2-(2-aminothiazol-4-yl)-(Z)-2-méthoxyimino acétique avec ledit DFCCS de formule XV

ou

le dérivé réactif de formule Ib,

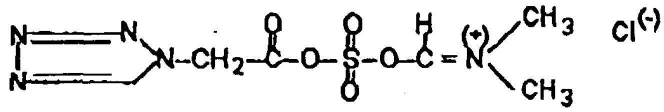

Ib

obtenu par réaction dudit acide 1H-tétrazol-1-acétique avec ledit DFCCS de formule XV

ou

un dérivé réactif de formule Ic

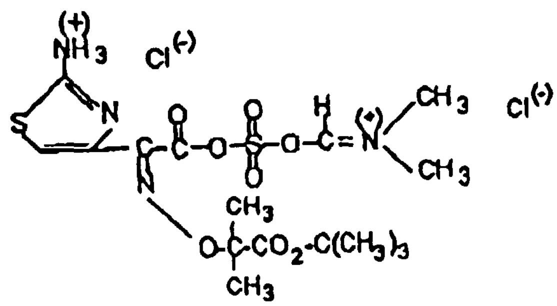

Ic

obtenu par réaction dudit acide 2-(2-aminothiazol-4-yl)-(Z)-2-[1-tertbutoxy carbonyl-1-méthyléthoxy)imino]acétique avec ledit DFCCS de formule XV.

5. Procédé selon la revendication 4 où le rapport molaire dudit DFCCS(XV) par rapport audit acide de 2-(2-aminothiazol-4-yl)-(Z)-2-[1-méthoxyimino acétique/ledit acide 1H-tétrazol-1-acétique est de 1,1 à 1,2.

6. Procédé selon la revendication 5 où le rapport molaire dudit DFCCS(XV) par rapport audit acide 2-(2-aminothiazol-4-yl)-(Z) -2-méthoxyimino acétique/ledit acide 1H-tétrazol-1-acétique est de 1,17.

7. Procédé selon la revendication 4 où le rapport molaire de DFCCS (XV) audit acide 2- (2-aminothiazol-4-yl) (Z)-2-[1-tertbutoxycarbonyl-1-méthyléthoxy)imino]acétique (V) est de 1,0 à 1,3.

8. Procédé pour la préparation d'antibiotiques de céphalosporine de la formule II

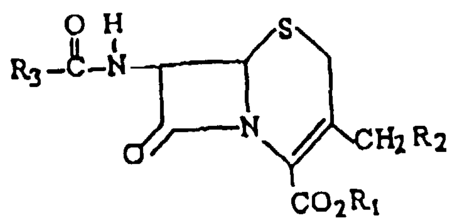

II

où R1 = H, un groupe carboxylique protecteur

IIa : $R_2$ = acétoxy , $R_3$ = 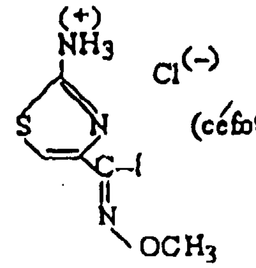 (céfotaxime)

IIb : R = 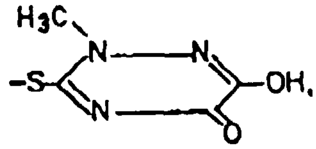 , $R_3$ = 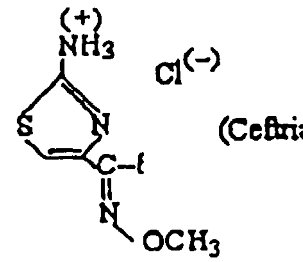 (Ceftriaxone)

qui comprend la réaction de dérivé silylé de l'acide 7-amino céphalosporanique de formule XVI

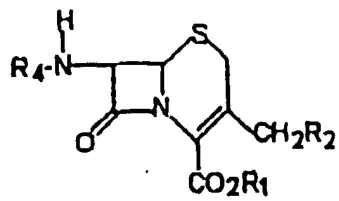

XVI

où R1, R2 est tel que défini dans ladite formule II ; R4 = groupe silyle ou hydrogène avec un dérivé réactif de formule Ia dans le dichlorométhane, le chloroforme, le benzène ou le toluène à une température comprise entre -70°C et -30°C.

**9.** Procédé pour la préparation d'antibiotiques de céphalosporine de formule II
où R1 = H, un groupe carboxylique protecteur

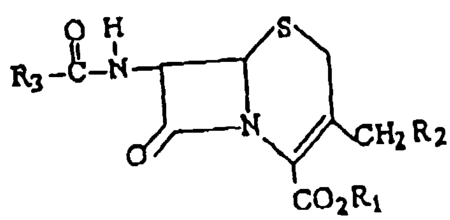

II

IIc : $R_2$ = 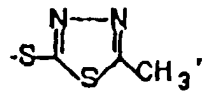, $R_3$ = 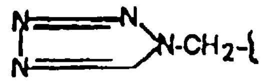 (céfazoline)

qui comprend la réaction de dérivé silylé de l'acide 7-amino céphalosporanique de formule XVI,

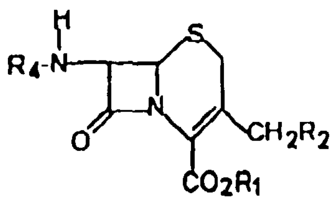

XVI

où R1, R2 est tel que défini dans ladite formule II ; R4 = groupe silyle ou hydrogène ; avec un dérivé réactif de formule Ib dans le dichlorométhane, le chloroforme, le benzène ou le toluène à une température comprise entre -70°C et -30°C.

**10.** Procédé selon l'une quelconque des revendications 8 ou 9 où la température va de -70°C à -65°.

**11.** Procédé pour la préparation de ceftazidime de formule IId

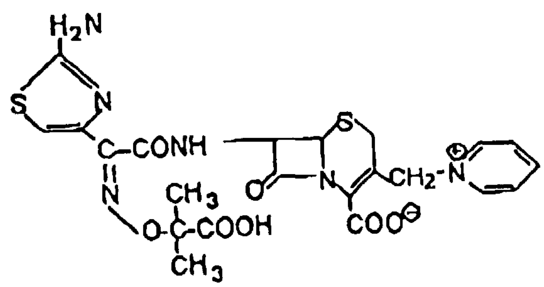

IId

qui comprend

a. la silylation de l'acide 7-amine-3-(1-pyridiniumméthyl)-3-céphèm-4-carboxylique de formule VII,

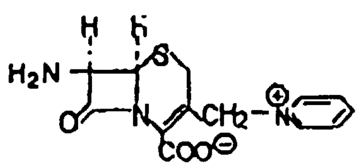

VII

avec un agent silylant en présence d'un agent balayant les acides à une température allant de 18°C à 25°C pour produire l'acide 7-amino-3-(1-pyridiniumméthyl)-3-céphém-4-carboxylique de formule XVId

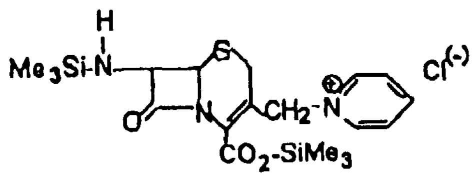

XVId

b. l'activation de l'acide 2-(2-aminothiazol-4-yl)-(Z)-2-[(1-tertbutoxycarbonyl-1-méthyléthoxy)imino]acétique (V)

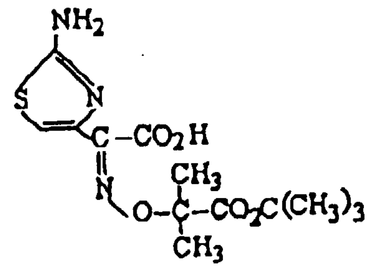

V

avec du chlorure de N,N-diméthylformiminium chlorosulfate (DFCCS)XV

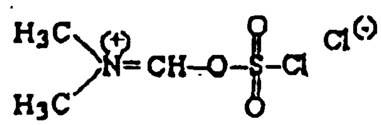

XV

dans le dichlorométhane à une température allant de -20°C à -25°C pour produire le dérivé réactif de formule Ic ;

c. l'acylation de l'acide 7-amino-3-(1-pyridiniumméthyl) -3-céphèm-4-carboxylique (XVId) silylé de l'étape (a) avec le dérivé réactif de formule IC de l'étape (b) à une température allant de -70°C à -60°C pour obtenir le tertbutyl ester de ceftazidime (XII),

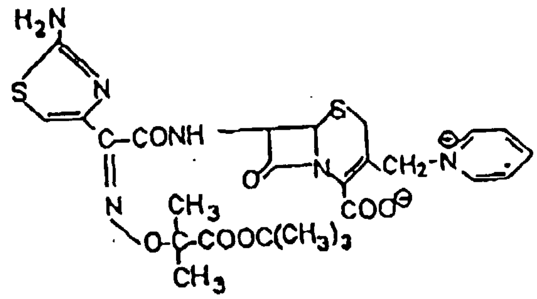

XII

qui est converti en ceftazidine pentahydratée IId via le dichlorhydrate de ceftazidime.

**12.** Procédé selon la revendication 11, où l'acide 7-amino-3-(1-pyridiniumméthyl)-3-céphém-4-carboxylique silylé (XVId) est mis à réagir avec ledit dérivé réactif de formule IC en présence d'un agent balayant les acides.

**13.** Procédé selon la revendication 11 ou 12, où ledit agent balayant les acides est sélectionné parmi la N,N-diméthylaniline, la diéthylamine, la pyridine.

**14.** Procédé selon l'une quelconque des revendications 11 à 13 où l'agent silylant est sélectionné parmi le N,O-bis(triméthylsilyl)acétamide, l'héxaméthyldisilazane, le triméthyl chlorosilane de préférence le N,O-bis(triméthylsilyl)acétamide.

**15.** Procédé selon l'une quelconque des revendications il à 14, où le rapport molaire de DFCCS (XV) à l'acide (Z)-2-(2-aminochiazol-4-yl)-2-[(1-certbutoxycarbonyl-1-méthyléthoxy)imino]acétique (V) est de 1,0 à 1,3.

**16.** Procédé selon l'une quelconque des revendications 8 à 15, où le rapport molaire dudit dérivé réactif de formule IA ou 1B ou IC par rapport audit 7-ACA silylé est de 1,1 à 2.

**17.** Procédé selon la revendication 16 où le rapport molaire dudit dérivé de la réaction de formule IA ou 1B ou 1C par rapport audit 7-ACA silylé est de 1,2.

**18.** Procédé pour la préparation de DFCCS de formule XV comprenant la réaction du diméthyl formamide (DMF) avec du chlorure de sulfuryle.

**19.** Procédé selon la revendication 18, où ledit DFCCS de formule XV est préparé par réaction d'une quantité équimolaire de chlorure de sulfuryle et de diméthyl formamide.

**20.** Procédé selon la revendication 18 ou 19 où ledit chlorure de N-VI-diméthylformiminium chlorosulfaté (DFCCS) de formule utilisé est obtenu par réaction dudit N,N-diméchylformamide (DMF) et dudit chlorure de sulfuryle en présence de 0,5 à 0,7 volume de dichlorométhane comme solvant.